# EUROPEAN PATENT APPLICATION

(11) **EP 4 311 858 A1**
(43) Date of publication of application: **31.01.2024**
(21) Application number: 22186956.3
(22) Date of filing: 26.07.2022
(51) Int. Cl.: C12N 15/63

(54) **INTEIN-BASED CONTROLLERS**

(71) Applicant: ETH Zurich, 8092 Zürich (CH)
(72) Inventor: ANASTASSOV, Stanislav, 8092 Zürich (CH); GEORGES, Maurice, 8092 Zürich (CH); CHANG, Ching-Hsiang, 8092 Zürich (CH); KHAMMASH, Mustafa Hani, 8092 Zürich (CH)
(74) Representative: Schulz Junghans Patentanwälte PartGmbB

(57) **Abstract**

The present invention relates to an expression system and a method ensuring constant-level concentration of an output. The invention also relates to a cell comprising the expression system.

## Description

### Field

The present invention relates to an expression system and a method ensuring constant-level concentration of an output. The invention also relates to a cell comprising the expression system.

### Background

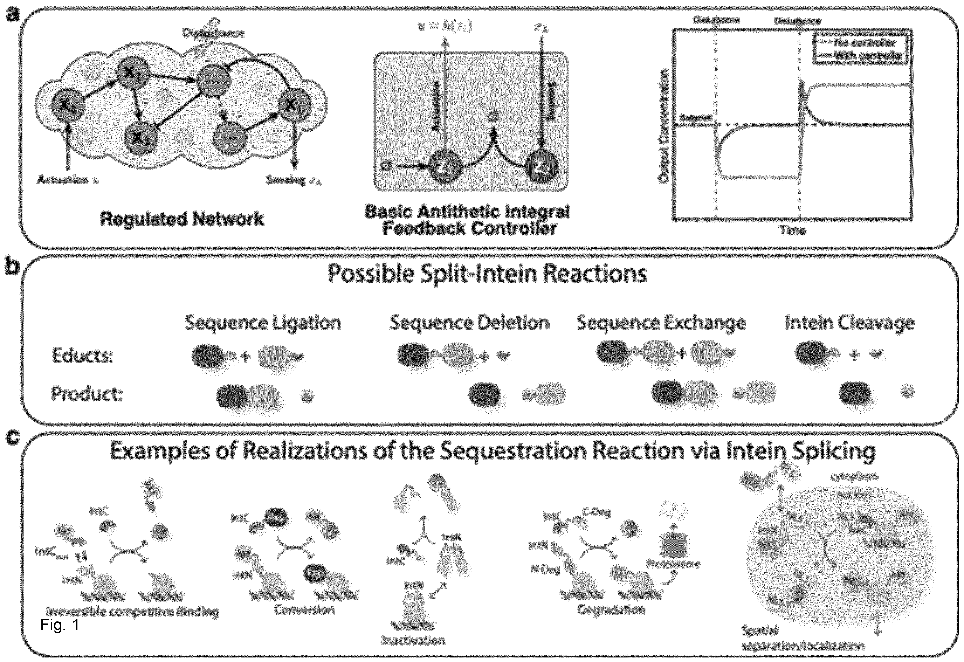

One of the essential features of living systems is their ability to maintain a robust behavior despite disturbances coming from their external uncertain and noisy environments. This feature, in pure biological terms, is referred to as **homeostasis** which is typically achieved via endogenous feedback regulatory mechanisms shaped by billions of years of evolution inside the cells. Pathological diseases are often linked to loss of homeostasis. The need to restore homeostasis for preventing such diseases, when endogenous regulatory mechanisms fail, was one of the main drivers of ushering a new active field of research referred to as cybergenetics - a field that brings control theory and synthetic biology together. In particular, the rational design of biomolecular feedback controllers offers promising candidates that may accompany or even replace such failed mechanisms (C. Kemmer et al., Nature biotechnology, vol. 28, no. 4, p. 355, 2010.; K. Rössger et al.," Nature communications, vol. 4, no. 1, pp. 1-9, 2013; M. Xie et al., Science, vol. 354, no. 6317, pp. 1296-1301, 2016). A notion which is similar to homeostasis, but more stringent, is **Robust Perfect Adaptation** (RPA) (F. Xiao and J. C. Doyle, IEEE, 2018, pp. 4345-4352; M. H. Khammash, Cell Systems, vol. 12, no. 6, pp. 509-521, 2021) which is the biological analogue of the well-known notion of robust steady-state tracking in control theory. A controller succeeds in achieving RPA if it drives the steady state of a variable of interest to a prescribed set-point despite varying initial conditions, plant uncertainties and/or constant disturbances. Motivated by the internal model principle (B. A. Francis and W. M. Wonham, Automatica, vol. 12, no. 5, pp. 457-465, 1976.), which establishes that the designed controller must implement an integral feedback component to be able to achieve RPA, the **antithetic integral controller** (C. Briat et al., Cell Systems, vol. 2, no. 1, pp. 15-26, 2016) was brought forward (see Fig. 1(a)).

The task of building genetic controllers can be divided into two sub-tasks. First, one has to design the **reaction network topologies** that are capable of mathematically realizing integral control structures that achieve RPA, or even more advanced structures such as proportional-integral-derivative (PID) controllers that are capable of shaping the transient response and enhancing the performance while maintaining RPA. Then, one has to find the **genetic parts** whose dynamics are captured by the designed reaction network topologies. The inventors have proposed, analyzed and built several reaction network topologies and genetic parts that realize partial or full PID biomolecular controllers in the inventors' previously filed patent application and the inventors' published papers (M. Filo, S. Kumar, and M. Khammash, Nature Communications, vol. 13, no. 1, pp. 1-19, 2022; S. K. Aoki et al., Nature, p. 1, 2019; T. Frei et al., PNAS, 2022, 119 (24)).

Based on the above-mentioned state of the art, the objective of the present invention is to provide means and methods for an expression system capable of ensuring constant-level concentration of an output protein. This objective is attained by the subject-matter of the independent claims of the present specification, with further advantageous embodiments described in the dependent claims, examples, figures and general description of this specification.

### Summary of the Invention

In the inventors' current work, the inventors use new genetic parts based on inteins to build genetic controllers capable of achieving RPA. These new genetic parts also give rise to a broad class of new reaction network topologies.

An intein is a segment of a protein that is capable of autocatalytically cutting itself from the protein and reconnecting the remaining segments, called exteins, via peptide bonds. Inteins are universal as they can be naturally found in all domains of life spanning eukaryotes, bacteria, archaea and viruses. Split-inteins - a subset class of inteins - are, as the name suggests, inteins split into two halves commonly referred to as IntN and IntC. These small protein segments are capable of heterodimerizing and performing protein splicing reactions on their own where they break and form new peptide bonds. These features can be exploited to ligate, remove or exchange amino acid sequences (see Fig. 1(b)). The individual halves can be as small as a few dozen of amino acids and, as a result, their production requires little amount of cellular resources. Split-inteins have been widely studied and characterized due to their extensive usage in various life science disciplines. In fact, they are typically used for fast, reliable and irreversible posttranslational modifications. Furthermore, they naturally exist as orthogonal pairs with different kinetic properties, where each pair react among each other without interfering with other pairs. The inventors shall refer to these reactions as "intein-splicing reactions" where molecules containing IntC react with molecules containing IntN to undergo a particular splicing mechanism. Note that split inteins are labeled as active when they are capable of undergoing an intein-splicing reaction; whereas inactive intein complexes denote hetero-dimerized IntC-IntN molecules that are incapable of undergoing any intein-splicing reactions. When two molecules undergo an intein-splicing reaction, the IntN and IntC segments are stoichiometrically inactivated; however the products of such a reaction may still have other functions such as activating or repressing gene expression.

The basic idea of the inventors' work is to leverage the flexibility offered by split-inteins to design biomolecular controllers capable of achieving Robust Perfect Adaptation (RPA). This flexibility is mainly a consequence of their compatibility with essentially any transcription factor. In fact, the particular structure of the expressed transcription factor involving the choices of the activation domain, dimerization domain, DNA-binding domain and insertion position of the split intein opens the possibilities to a broad spectrum of controllers (see Fig. 1(c)). In fact, this is true not only for transcription factors, but also for other classes of proteins. For instance, it is possible to insert a split-intein between the transmembrane domains of a cytoplasmic domain and a receptor. The resulting intein-splicing reaction would lead to the truncation of the cytoplasmic domain hence preventing signal transduction into the cell.

A first aspect of the invention relates to a method for constant-level expression of an output. The method comprises the steps:
a. providing a cell or a cell-free system, wherein the cell or the cell-free system is capable of expressing a gene encoding the output;
b. inserting an expression system into said cell or cell-free system,
c. exposing said cell or cell-free system to a condition, wherein the expression level of the output is perturbed from a target level to a perturbed level;
d. awaiting an equilibration of the expression level of the output back to the target level.

A second aspect of the invention relates to an expression system for constant-level expression of an output.

A third aspect of the invention relates to a cell comprising the expression system according to the second aspect.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of' or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

Any patent document cited herein shall be deemed incorporated by reference herein in its entirety.

### General Biochemistry: Peptides, Amino Acid Sequences

The term *polypeptide* in the context of the present specification relates to a molecule consisting of 50 or more amino acids that form a linear chain wherein the amino acids are connected by peptide bonds. The amino acid sequence of a polypeptide may represent the amino acid sequence of a whole (as found physiologically) protein or fragments thereof. The term "polypeptides" and "protein" are used interchangeably herein and include proteins and fragments thereof. Polypeptides are disclosed herein as amino acid residue sequences.

The term *peptide* in the context of the present specification relates to a molecule consisting of up to 50 amino acids, in particular 8 to 30 amino acids, more particularly 8 to 15 amino acids, that form a linear chain wherein the amino acids are connected by peptide bonds.

Amino acid residue sequences are given from amino to carboxyl terminus. Capital letters for sequence positions refer to L-amino acids in the one-letter code (Stryer, Biochemistry, 3^{rd} ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (GIn, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

In the context of the present specification, the term *dimer* refers to a unit consisting of two subunits.

### General Molecular Biology: Nucleic Acid Sequences, Expression

The term *gene* refers to a polynucleotide containing at least one open reading frame (ORF) that is capable of encoding a particular polypeptide or protein after being transcribed and translated. A polynucleotide sequence can be used to identify larger fragments or full-length coding sequences of the gene with which they are associated. Methods of isolating larger fragment sequences are known to those of skill in the art.

The terms *gene expression* or *expression,* or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of a RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

The term *nucleic acid expression vector* in the context of the present specification relates to a plasmid, a viral genome or an RNA, which is used to transfect (in case of a plasmid or an RNA) or transduce (in case of a viral genome) a target cell with a certain gene of interest, or -in the case of an RNA construct being transfected- to translate the corresponding protein of interest from a transfected mRNA. The term *nucleic acid expression vector* in the context of the present specification also relates to a plasmid or an RNA in a cell-free system. For vectors operating on the level of transcription and subsequent translation, the gene of interest is under control of a promoter sequence and the promoter sequence is operational inside the target cell or the cell-free system, thus, the gene of interest is transcribed either constitutively or in response to a stimulus or dependent on the cell's or system's status. In certain embodiments, the viral genome is packaged into a capsid to become a viral vector, which is able to transduce the target cell.

In the context of the present specification, the term *expression system* refers to a nucleic acid-based encoding of certain genes in a context which allows for expression of the genes. In certain embodiments, each gene of the expression system is preceded (in 5' to 3' direction) by a promoter. In certain embodiments, the expression system is composed of one or several expression vectors. In certain embodiments, the expression system is composed of one or several plasmids. In certain embodiments, the expression system is encoded in the genome of the target cell. In certain embodiments, the expression system encoded in a viral particle which then is used to transform the target cell. In certain embodiments, the expression system is composed of DNA. In certain embodiments, the expression system is composed of RNA. In certain embodiments, the expression system additionally comprises enhancer sequences or other regulatory sequences.

In the context of the present specification, the term *upregulating the concentration* refers to a mechanism or a signal cascade which leads to higher amount of the target polypeptide or peptide (termed "target" hereafter) than the amount which would have been produced without the agent that upregulates the concentration. In certain embodiments, the concentration is upregulated via an increased expression of the target. In certain embodiments, the concentration is upregulated via increased transcription of the target. In certain embodiments, the concentration is upregulated via increased translation of the target. In certain embodiments, the concentration is upregulated via decreased degradation of the target. In certain embodiments, the concentration is upregulated via activation of the target. In certain embodiments, this activation is achieved via a posttranslational modification of the target. In certain embodiments, the concentration is upregulated directly by the agent that upregulates the concentration with the agent being a transcription factor, and the target being under control of that transcription factor. In certain embodiments, the concentration is upregulated via formation of a target protein complex. In certain embodiments, the concentration is upregulated via a combination of the mechanisms described before.

In the context of the present specification, the term *downregulating the concentration* refers to a mechanism or a signal cascade which leads to lower amount of the target polypeptide or peptide (termed "target" hereafter) than the amount which would have been produced without the agent that downregulates the concentration. In certain embodiments, the concentration is downregulated via a decreased expression of the target. In certain embodiments, the concentration is downregulated via decreased transcription of the target. In certain embodiments, the concentration is downregulated via decreased translation of the target. In certain embodiments, the concentration is downregulated via increased degradation of the target. In certain embodiments, the concentration is downregulated via inactivation of the target. In certain embodiments, this inactivation is achieved via a posttranslational modification of the target. In certain embodiments, the concentration is downregulated directly by the agent that downregulates the concentration with the agent being a transcription repressor, and the target being under control of that transcription repressor. In certain embodiments, the concentration is downregulated via sequestration of a target protein complex. In certain embodiments, the concentration is downregulated via a combination of the mechanisms described before.

In the context of the present specification, the terms *upregulating or downregulating the concentration* do not necessarily mean that all mentioned agents only have the respective effect. It rather means that the summed effect of all mentioned agents under steady-state conditions have the respective effect.

As an example, if the second and the optional first splice-product downregulate the output, it could be that an intermediate splice-product has an upregulating effect on the output. It could also be that e.g. the first splice-product has an upregulating effect, but the second splice-product has an even stronger downregulating effect. It could also be that a single species, as e.g. the first splice-product, has a mixed effect on the concentration of the output, and partially upregulates and partially downregulates the concentration of the output. Yet, in this example, the summed effect of the first and the second splice-product under steady-state conditions is an upregulation of the output.

In the context of the present specification, the term *steady-state condition* refers to the maintenance of constant internal concentrations of molecules and ions. A continuous flux of mass and energy results in the constant synthesis and breakdown of molecules via chemical reactions of biochemical pathways. In the present context, steady-state refers to a situation in which the output expression level is not disturbed via an external stimulus, or in which the disturbance has already been re-equilibrated.

In the context of the present specification, the term *effector* refers to a peptide or polypeptide which (optionally in combination with other effectors) has an impact or influence on the concentration of a target protein, i.e. the output. In certain embodiments, each effector (which is present) is composed of or comprises one or several protein domain(s). In certain particular embodiments, each effector (which is present) comprises one or several transcription factor domain(s). Before the trans-splicing reaction of the N-intein and the C-intein occurs, the first and the second effectors are in close proximity, and the third and the fourth effectors are in close proximity. After the trans-splicing reaction of the N-intein and the C-intein has occurred, the first and the fourth effectors are in close proximity, and the second and the third effectors are in close proximity. Depending on their proximity, the different domains interact in a different way. In certain embodiments, several trans-splicing reactions occur via several N-inteins and/or several C-inteins, but the same rules as for single inteins apply.

In the context of the present specification, the term *intein* refers to a split-intein. Split inteins are split into two fragments called N-intein (I_{N}) and C-intein (I_{C}). Once translated, the intein fragments bind to each other allowing them to assemble intermolecularly into a fully functional protein splicing unit, which catalyzes protein splicing in trans. For further details see Sarmiento and Camarero (Curr Protein Pept Sci. 2019; 20(5): 408-424.).

In the context of the present specification, the term *trans-splicing* refers to excision of the N-intein and the C-intein from their polypeptide chain, and rejoining of the (poly)peptide N-terminal of the N-intein with the (poly)peptide C-terminal of the C-intein. The N-intein forms a non-covalent protein complex with the C-intein when brought in close proximity. This protein complex performs the trans-splicing reaction.

In the context of the present specification, the term *rejoining* refers to the formation of a covalent peptide bond. An N-terminal amino acid of one protein is linked to a C-terminal amino acid of the other protein via an amide bond between the protein backbones of these proteins.

In the context of the present specification, the term *perturbation* refers to a deviation of a concentration level from the target level to a perturbed level. In certain embodiments, an external stimulus leads to the perturbation of the expression level.

In the context of the present specification, the term *cell* refers to a prokaryotic cell or a eukaryotic cell.

In the context of the present specification, the term *cell-free system* refers to an *in vitro* system derived from a prokaryotic cell or a eukaryotic cell, wherein the system contains the native transcription, translation, and metabolic machineries required to achieve gene expression.

In the context of the present specification, the term *loop region* refers to a longer, extended or irregular loop inside a protein structure without fixed internal hydrogen bonding.

In the context of the present invention, the second splice-product and the optional first splice-product have a weaker, neutral, or opposite effect on the concentration of the output in comparison to the effect of the controller. This means that if
a. the controller upregulates the concentration of the output:
   The second splice-product and the optional first splice-product in sum do not upregulate the concentration of the output to the same extent as the controller does. Thus, the second splice-product and the optional first splice-product in sum
   i. weakly upregulate the concentration of the output (significantly weaker than the controller upregulates the output); or
   ii. have no (a neutral) effect on the concentration of the output; or
   iii. downregulate the concentration of the output.
b. the controller downregulates the concentration of the output:
   The second splice-product and the optional first splice-product in sum do not downregulate the concentration of the output to the same extent as the controller does. Thus, the second splice-product and the optional first splice-product in sum
   i. weakly downregulate the concentration of the output (significantly weaker than the controller downregulates the output); or
   ii. have no (a neutral) effect on the concentration of the output; or
   iii. upregulate the concentration of the output.

This feature of the second splice-product and the optional first splice-product is important in achieving perfect-robust-adaption. The idea is that the controller has a certain effect on the concentration of the output, and that this effect is lowered or even reversed when the controller reacts with the anti-controller to form the second splice-product and the optional first splice-product.

The step of *awaiting an equilibration of the expression level of the output back to the target level* is to be understood as follows: The proposed intein-based controller circuits realize what is in control theory referred to as "Integral Feedback Control" - one of the most predominant control strategies in industry. This type of feedback is more stringent than simple negative feedback where the controller's task is to only oppose the variation of the output of interest to be controlled. In contrast, the integral feedback controller, realized with split inteins, determines the error or, more specifically, the deviation of the output from the desired setpoint and feeds back the mathematical integral as the control action that steers the output to the desired level. It is precisely this mathematical determination of the integral of the error that endows the system with the robust perfect adaptation property that guarantees the equilibration of the output concentration level back to its pre-perturbed state. These mathematical determinations are encoded in the various reactions occurring between the controller and anti-controller species. In certain embodiments, the controller is constitutively expressed to encode for the desired setpoint level. In certain embodiments, the expression of the anti-controller is induced by the output to "sense" its abundance. In certain embodiments, the intein-splicing reaction between the controller and anti-controller encodes for the comparison between setpoint and output concentration to compute the error and its mathematical integral. In certain embodiments, the controller molecules that are not sequestered by the anti-controller induces the expression of the output, and thus effectively closes the loop by feeding back the mathematical integral. In certain embodiments, the spliced products aid in enhancing the transient dynamic performance (e.g. speeding up the response, damping oscillations, reducing overshoots, etc.).

### Detailed Description of the Invention

A first aspect of the invention relates to a method for constant-level expression of an output. The method comprises the steps:
a. providing a cell or a cell-free system, wherein the cell or the cell-free system is capable of expressing a gene encoding the output under control of a first promoter;
b. inserting an expression system into said cell or cell-free system,
c. exposing said cell or cell-free system to a condition, wherein the expression level of the output is perturbed from a target level to a perturbed level;
d. awaiting an equilibration of the expression level of the output back to the target level.

The first, second, third, and fourth effector are peptides or polypeptides, and the first and second intein, the controller, and the anti-controller are polypeptides. The output is a peptide, a polypeptide, or an mRNA.

A second aspect of the invention relates to an expression system for constant-level expression of an output which is under control of a first promoter.

The expression system of the first and the second aspect comprises:
i. a gene encoding a controller under control of a second promoter, wherein the controller comprises from N to C-terminus:
   I. an optional first effector;
   II. a first split-intein; and
   III. an optional second effector;
ii. a gene encoding an anti-controller under control of a third promoter, wherein the anti-controller comprises from N to C-terminus:
   I. an optional third effector;
   II. a second split-intein; and
   III. an optional fourth effector.

Thus, the controller comprises
a) a first effector, a first split-intein, and a second effector; or
b) a first effector, and a first split-intein; or
c) a first split-intein, and a second effector; or
d) only a first split-intein,
and, the anti-controller comprises
i.) a third effector, a second split-intein, and a fourth effector; or
ii.) a third effector, and a second split-intein; or
iii.) a second split-intein, and a fourth effector; or
iv.) only a second split-intein;
with the proviso that at least one of the optional effectors is present (the option of d) the controller comprises only a first split-intein cannot be combined with the option of iv.) the anti-controller comprises only a second split-intein).

One of the first split-intein and the second split-intein is an N-intein and the other one is a C-intein. Thus, either the first split-intein is an N-intein, and the second split-intein is a C-intein, or the first split-intein is a C-intein, and the second split-intein is an N-intein.

The controller and the anti-controller when being in close proximity are capable of undergoing a trans-splicing reaction. The trans-splicing reaction depending on whether the first split-intein (alternative a) or the second split-intein (alternative b) is the N-intein comprises the following steps:
*Alternative a): the first split-intein is an N-intein, and the second split-intein is a* C-*intein:*
   The first split-intein is excised from the controller, and the second split-intein is excised from the anti-controller. The first effector if present is rejoined with the fourth effector if present yielding a first splice-product. Thus, the first splice-product consists of
   - the first effector, and the fourth effector, or
   - only of the first effector, or
   - only of the fourth effector, or
   - is not present at all.
   A non-covalently associated protein complex is assembled consisting of the N-intein covalently bound to the second effector if present with the C-intein covalently bound to the third effector if present yielding a second splice-product. Thus, the second splice-product is a non-covalently associated protein complex which consists of
   - the N-intein covalently bound to the second effector, and the C-intein covalently bound to the third effector, or
   - the N-intein covalently bound to the second effector, and the C-intein,
   - the N-intein, and the C-intein covalently bound to the third effector,
   - the N-intein, and the C-intein.
   The only proviso is that one of the optional effectors is present, thus if the first splice-product is not present at all, the second splice-product cannot consist only of the N-intein and the C-intein.
*Alternative b): the first split-intein is a C-intein, and the second split-intein is an N-intein:*
   The first split-intein is excised from the controller, and the second split-intein is excised from the anti-controller. The second effector if present is rejoined with the third effector if present yielding a first splice-product. Thus, the first splice-product consists of
   - the second effector, and the third effector, or
   - only of the second effector, or
   - only of the third effector, or
   - is not present at all.
   A non-covalently associated protein complex is assembled consisting of the N-intein covalently bound to the fourth effector if present with the C-intein covalently bound to the first effector if present yielding a second splice-product. Thus, the second splice-product is a non-covalently associated protein complex which consists of
   - the N-intein covalently bound to the fourth effector, and the C-intein covalently bound to the first effector, or
   - the N-intein covalently bound to the fourth effector, and the C-intein,
   - the N-intein, and the C-intein covalently bound to the first effector,
   - the N-intein, and the C-intein.
   The only proviso is that one of the optional effectors is present, thus if the first splice-product is not present at all, the second splice-product cannot consist only of the N-intein and the C-intein.

The anti-controller is capable of downregulating the controller stoichiometrically via undergoing the trans-splicing reaction. This is because the controller and the anti-controller react in the trans-splicing reaction yielding the second splice-product and optionally the first splice-product.

The second splice-product and the optional first splice-product have a weaker, neutral, or opposite effect on the concentration of the output in comparison to the effect of the controller. Thus, the effect of the controller on the output is decreased or even reversed when the controller reacts with the anti-controller.

Constant-level expression of the output is achieved via one of the following four alternatives of a steady-state route (also called architecture or topology).

### Alternative (1) of a steady-state route:

The controller upregulates or is capable of upregulating the concentration of the output. The output upregulates or is capable of upregulating the concentration of the anti-controller. The second splice-product and the optional first splice-product are not capable of upregulating the concentration of the output to the extent as the controller (weaker upregulation, neutral effect, or downregulation). In certain embodiments, the anti-controller additionally downregulates or is capable of downregulating the concentration of the output.

### Alternative (2) of a steady-state route:

The controller downregulates or is capable of downregulating the concentration of the output. The output downregulates or is capable of downregulating the concentration of the anti-controller. The second splice-product and the optional first splice-product are not capable of downregulating the concentration of the output to the extent as the controller (weaker downregulation, neutral effect, or upregulation). In certain embodiments, the anti-controller additionally upregulates or is capable of upregulating the concentration of the output.

### Alternative (3) of a steady-state route:

The controller upregulates or is capable of upregulating the concentration of the output. The output downregulates or is capable of downregulating the concentration of the controller. The second splice-product and the optional first splice-product are not capable of upregulating the concentration of the output to the extent as the controller (weaker upregulation, neutral effect, or downregulation). In certain embodiments, the anti-controller additionally downregulates or is capable of downregulating the concentration of the output.

### Alternative (4) of a steady-state route:

The controller downregulates or is capable of downregulating the concentration of the output. The output upregulates or is capable of upregulating the concentration of the controller. The second splice-product and the optional first splice-product are not capable of downregulating the concentration of the output to the extent as the controller (weaker downregulation, neutral effect, or upregulation). In certain embodiments, the anti-controller additionally upregulates or is capable of upregulating the concentration of the output.

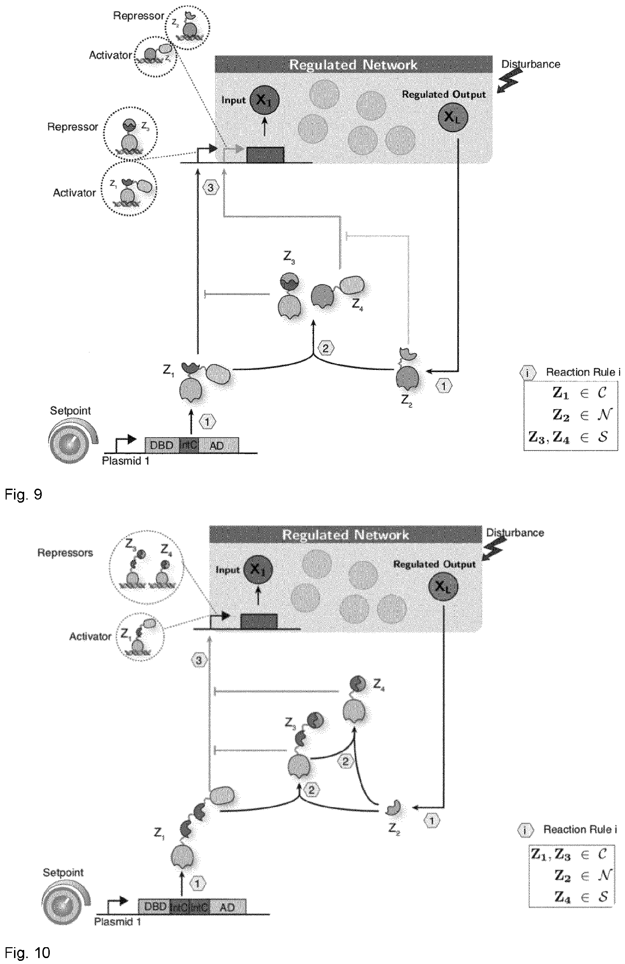

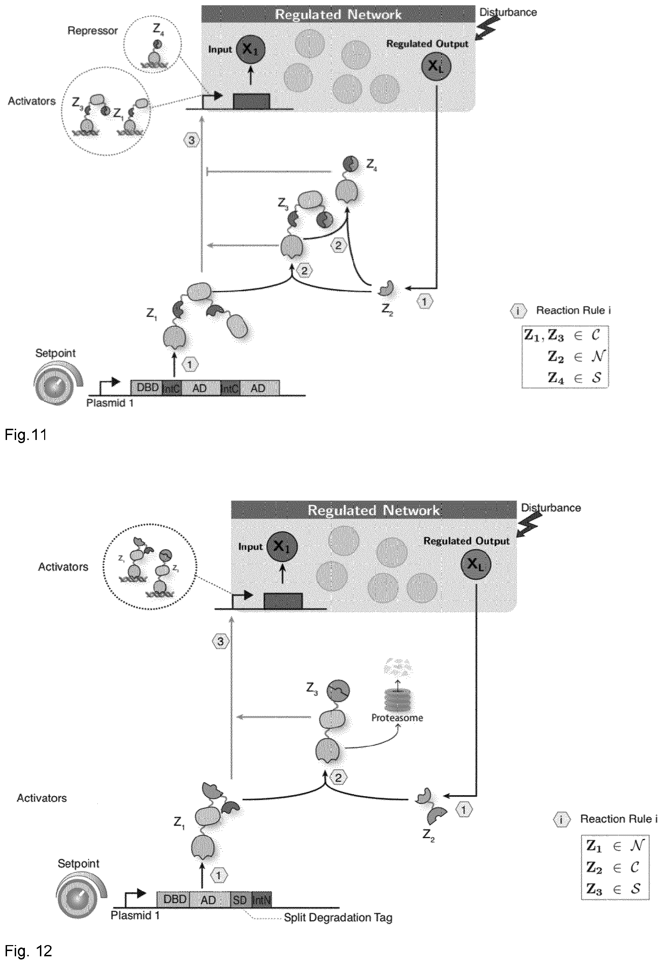

In certain embodiments, the controller comprises a further first intein and/or the anti-controller comprises a further second intein and the trans-splicing reaction is executed (or is executable) multiple times finally yielding the second splice-product and optionally the first splice-product. For an example, see Figs. 10 and 11.

In certain embodiments, the first intein is inserted into the controller in a loop region of the first and second effector. In certain embodiments, the second intein is inserted into the anti-controller in a loop region of the third and fourth effector.

In certain embodiments,
- the first effector, and/or
- the second effector, and/or
- the third effector, and/or
- the fourth effector
comprise a (one or several) domain(s) of a transcription factor. In certain embodiments, the first effector comprises a (one or several) domain(s) of a transcription factor. In certain embodiments, the second effector comprises a (one or several) domain(s) of a transcription factor. In certain embodiments, the third effector comprises a (one or several) domain(s) of a transcription factor. In certain embodiments, the fourth effector comprises a (one or several) domain(s) of a transcription factor.

In certain embodiments,
- the controller is a non-dimerizing transcription factor (TF) (particularly a zinc-finger-based transcription factor) modified by insertion of a C-intein;
- the first effector is a DNA-binding domain of the TF;
- the second effector is an activation domain of the TF;
- the anti-controller is an N-intein;
- the third and the fourth effectors are not present.

In certain embodiments,
- the controller is a dimerizing transcription factor (TF) (particularly a tetR-based transcription factor) modified by insertion of a C-intein;
- the first effector consists of
   ∘ a DNA-binding domain of the TF;
   ∘ an N-terminal part of a dimerization domain of the TF;
- the second effector consists of
   ∘ a C-terminal part of a dimerization domain of the TF;
   ∘ an activation domain of the TF;
- the anti-controller is an N-intein;
- the third and the fourth effectors are not present.

In certain embodiments,
- the controller is a dimerizing transcription factor (TF) (particularly a tTA-based transcription factor) modified by insertion of a C-intein;
- the first effector consists of
   ∘ a DNA-binding domain of the TF;
   ∘ a dimerization domain of the TF;
- the second effector is an activation domain of the TF;
- the anti-controller is an N-intein;
- the third and the fourth effectors are not present.

In certain embodiments,
- the controller is a dimerizing transcription factor (TF) (particularly a GAL4-based transcription factor) modified by insertion of a C-intein;
- the first effector is a DNA-binding domain of the TF;
- the second effector consists of
   ∘ a dimerization domain of the TF;
   ∘ an activation domain of the TF;
- the anti-controller is an N-intein;
- the third and the fourth effectors are not present.

In certain embodiments,
- the controller is an activation domain modified by fusion of a C-intein;
- the second effector is an activation domain;
- the first effector is not present;
- the anti-controller is an N-intein;
- the third and the fourth effectors are not present;
- the expression system additionally comprises a co-controller, wherein the co-controller comprises from N-terminus to C-terminus
   ∘ a DNA-binding domain of the TF;
   ∘ a dimerization domain of the TF;
   ∘ an inactive N-intein.

A third aspect of the invention relates to a cell comprising the expression system according to the second aspect.

Wherever alternatives for single separable features such as, for example, an isotype protein or ligand type or network topology are laid out herein as "embodiments", it is to be understood that such alternatives may be combined freely to form discrete embodiments of the invention disclosed herein. Thus, any of the alternative embodiments for an isotype protein may be combined with any of the alternative embodiments of ligand and these combinations may be combined with any network topology mentioned herein.

The invention further encompasses the following items:

### Items

0. A method for constant-level expression of an output, said method comprising the steps:
   a. providing a cell and/or a cell-free system, wherein the system is capable of acting positively or negatively on the levels of a specific output of interest, which can be part of a larger circuit (regulated network) inside and/or outside of said cell or cell-free system.
   b. inserting an expression controller system into said cell or cell-free system, providing closed-loop stability, wherein the expression controller system is composed of three subnetworks: N-class, C-class, and S-class.
      i. N-class.
         I. Every protein and protein complex in this subnetwork contains at least one functional IntN of a split-intein which can pair with a functional IntC from the C-class to undergo an intein-splicing reaction. The said IntNs can be attached to or embedded inside and/or between one or multiple effector molecules.
         II. At least one protein within this class is produced either constitutively or inducibly by the controlled output species. Their expression rates can be positively or negatively influenced by the controlled output species.
         III. Proteins and protein complexes within this class are allowed to be converted to one another while conserving the number of active IntN segments.
         IV. Proteins and protein complexes within this class are allowed to bind with other controller species while conserving the number of active IntN segments.
         V. At least one protein belongs to this class.
      ii. C-class
         I. Every protein and protein complex in this subclass contains at least one functional IntC of a split-intein which can pair with the functional IntN from the N class to undergo an intein-splicing reaction. The said IntCs can be attached to or embedded inside and/or between one or multiple effector molecules.
         II. At least one protein within this class is produced either constitutively or inducibly by the controlled output species. Their expression rates can be positively or negatively influenced by the controlled output species.
         III. Proteins and protein complexes within this class are allowed to be converted to one another while conserving the number of active IntC segments.
         IV. Proteins and protein complexes within this class are allowed to bind with other controller species while conserving the number of active IntN segments.
         V. At least one protein belongs to this class.
      iii. S-class
         I. All protein and protein complexes, which do not belong to the C- and N-classes, belong to the S-class.
1. A method for constant-level expression of an output, said method comprising the steps:
   a. providing a cell or a cell-free system, wherein the cell or the cell-free system is capable of expressing a gene encoding the output;
   b. inserting an expression system into said cell or cell-free system, wherein the expression system comprises:
      i. a gene encoding a controller, wherein the controller comprises:
         I. an optional first effector;
         II. a first split-intein; and
         III. an optional second effector;
      ii. a gene encoding an anti-controller, wherein the anti-controller comprises:
         I. an optional third effector;
         II. a second split-intein; and
         III. an optional fourth effector;
      wherein
      - at least one of the optional effectors is present;
      - one of the first split-intein and the second split-intein is an N-intein and the other one is a C-intein;
      - the controller and the anti-controller are capable of undergoing a trans-splicing reaction via the steps:
         - excising the first split-intein from the controller, and excising the second split-intein from the anti-controller, and
            a) if the first split-intein is an N-intein, and the second split-intein is a C-intein:
               - rejoining
                  ∘ the first effector if present with
                  ∘ the fourth effector if present
                  yielding a first splice-product;
               - assembling a protein complex of
                  ∘ the N-intein
                     ▪ bound to the second effector if present
                     with
                  ∘ the C-intein
                     ▪ bound to the third effector if present
                  yielding a second splice-product;
            b) if the first split-intein is a C-intein, and the second split-intein is an N-intein:
               - rejoining
                  ∘ the second effector if present with
                  ∘ the third effector if present
                  yielding a first splice-product;
               - assembling a protein complex of
                  ∘ the N-intein
                     ▪ bound to the fourth effector if present
                     with
                  ∘ the C-intein
                     ▪ bound to the first effector if present
                  yielding a second splice-product;
               wherein at least the second splice-product is yielded;
      - the anti-controller is capable of downregulating the controller stoichiometrically via undergoing the trans-splicing reaction;
      - the second splice-product and the optional first splice-product have a weaker, neutral, or opposite effect on the concentration of the output in comparison to the effect of the controller;
      - constant-level expression of the output is achieved via a route selected from the group consisting of:
         (1)
            - the controller upregulates the concentration of the output;
            - the output upregulates the concentration of the anti-controller;
         (2)
            - the controller downregulates the concentration of the output;
            - the output downregulates the concentration of the anti-controller;
         (3)
            - the controller upregulates the concentration of the output;
            - the output downregulates the concentration of the controller;
         (4)
            - the controller downregulates the concentration of the output;
            - the output upregulates the concentration of the controller;
   c. exposing said cell or cell-free system to a condition, wherein the expression level of the output is perturbed from a target level to a perturbed level;
   d. equilibration of the expression level of the output back to the target level;
   wherein the first, second, third, and fourth effector are peptides or polypeptides, and the first and second intein, the controller, and the anti-controller are polypeptides, and the output is a peptide, a polypeptide, or an mRNA.
2. An expression system for constant-level expression of an output, said system comprising:
   a. a gene encoding a controller, wherein the controller comprises:
      i.) an optional first effector;
      ii.) a first split-intein; and
      iii.) an optional second effector;
   b. a gene encoding an anti-controller, wherein the anti-controller comprises:
      i.) an optional third effector;
      ii.) a second split-intein; and
      iii.) an optional fourth effector;
   wherein
   - at least one of the optional effectors is present;
   - one of the first split-intein and the second split-intein is an N-intein and the other one is a C-intein;
   - the controller and the anti-controller are capable of undergoing a trans-splicing reaction via the steps:
   - excising the first split-intein from the controller, and excising the second split-intein from the anti-controller, and
      a) if the first split-intein is an N-intein, and the second split-intein is a C-intein:
         - rejoining
            ∘ the first effector if present with
            ∘ the fourth effector if present
            yielding a first splice-product;
         - assembling a protein complex of
            ∘ the N-intein
               ▪ bound to the second effector if present
               with
            ∘ the C-intein
               ▪ bound to the third effector if present
            yielding a second splice-product;
      b) if the first split-intein is a C-intein, and the second split-intein is an N-intein:
         - rejoining
            ∘ the second effector if present with
            ∘ the third effector if present
            yielding a first splice-product;
         - assembling a protein complex of
            ∘ the N-intein
               ▪ bound to the fourth effector if present
               with
            ∘ the C-intein
               ▪ bound to the first effector if present
            yielding a second splice-product;
         wherein at least the second splice-product is yielded;
   - the anti-controller is capable of downregulating the controller stoichiometrically via undergoing the trans-splicing reaction;
   - the second splice-product and the optional first splice-product have a weaker, neutral, or opposite effect on the concentration of the output in comparison to the effect of the controller;
   - a route via which constant-level expression of the output is achieved is selected from the group consisting of:
      (1)
         - the controller is capable of upregulating the concentration of the output;
         - the output is capable of upregulating the concentration of the anti-controller;
      (2)
         - the controller is capable of downregulating the concentration of the output;
         - the output is capable of downregulating the concentration of the anti-controller;
      (3)
         - the controller is capable of upregulating the concentration of the output;
         - the output is capable of downregulating the concentration of the controller;
      (4)
         - the controller is capable of downregulating the concentration of the output;
         - the output is capable of upregulating the concentration of the controller,
   wherein the first, second, third, and fourth effector are peptides or polypeptides, and the first and second intein, the controller, and the anti-controller are polypeptides, and the output is a peptide, a polypeptide, or an mRNA.
3. The method according to item 1 or the expression system according to item 2, wherein the controller comprises a further first intein and/or the anti-controller comprises a further second intein and the trans-splicing reaction is executed multiple times finally yielding the second splice-product and optionally the first splice-product.
4. The method according to item 1 or 3 or the expression system according to item 2 or 3, wherein
   - the controller is capable of upregulating the concentration of the output;
   - the second splice-product and the optional first splice-product are not capable of upregulating the concentration of the output to the extent as the controller;
   - the output is capable of upregulating the concentration of the anti-controller.
5. The method according to any one of the preceding items 1 or 3 to 4 or the expression system according to any one of the preceding items 2 to 4, wherein for case (1) that
   - the controller is capable of upregulating the concentration of the output;
   - the output is capable of upregulating the concentration of the anti-controller;
   additionally, the anti-controller is capable of downregulating the concentration of the output.
6. The method according to item 1 or 3 or the expression system according to item 2 or 3, wherein
   - the controller is capable of downregulating the concentration of the output;
   - the second splice-product and the optional first splice-product are not capable of downregulating the concentration of the output to the extent as the controller;
   - the output is capable of downregulating the concentration of the anti-controller.
7. The method according to any one of the preceding items 1 or 3 or 6 or the expression system according to any one of the preceding items 2 to 3 or 6, wherein for case (2) that
   - the controller is capable of downregulating the concentration of the output;
   - the output is capable of downregulating the concentration of the anti-controller;
   additionally, the anti-controller is capable of upregulating the concentration of the output.
8. The method according to item 1 or 3 or the expression system according to item 2 or 3, wherein
   - the controller is capable of upregulating the concentration of the output;
   - the second splice-product and the optional first splice-product are not capable of upregulating the concentration of the output to the extent as the controller;
   - the output is capable of downregulating the concentration of the controller.
9. The method according to any one of the preceding items 1 or 3 or 8 or the expression system according to any one of the preceding items 2 or 3 or 8, wherein for case (3) that
   - the controller is capable of upregulating the concentration of the output;
   - the output is capable of downregulating the concentration of the controller;
   additionally, the anti-controller is capable of downregulating the concentration of the output.
10. The method according to item 1 or 3 or the expression system according to item 2 or 3, wherein
   - the controller is capable of downregulating the concentration of the output;
   - the second splice-product and the optional first splice-product are not capable of downregulating the concentration of the output to the extent as the controller;
   - the output is capable of upregulating the concentration of the controller.
11. The method according to any one of the preceding items 1 or 3 or 10 or the expression system according to any one of the preceding items 2 or 3 or 10, wherein for case (4) that
   - the controller is capable of downregulating the concentration of the output;
   - the output is capable of upregulating the concentration of the controller;
   additionally, the anti-controller is capable of upregulating the concentration of the output.
12. The method according to any one of the preceding items 1 or 3 to 11 or the expression system according to any one of the preceding items 2 to 11, wherein the first intein is inserted into the controller in a loop region of the first and second effector and/or the second intein is inserted into the anti-controller in a loop region of the third and fourth effector.
13. The method according to any one of the preceding items 1 or 3 to 12 or the expression system according to any one of the preceding items 2 to 12, wherein
   - the first effector, and/or
   - the second effector, and/or
   - the third effector, and/or
   - the fourth effector
   comprise a domain of a transcription factor.
14. The method according to item 13 or the expression system according to item 13, wherein
   - the controller is a non-dimerizing transcription factor (TF) modified by insertion of a C-intein;
   - the first effector is a DNA-binding domain of the TF;
   - the second effector is an activation domain of the TF;
   - the anti-controller is an N-intein;
   - the third and the fourth effectors are not present.
15. The method according to item 13 or the expression system according to item 13, wherein
   - the controller is a dimerizing transcription factor (TF) modified by insertion of a C-intein;
   - the first effector consists of
      ∘ a DNA-binding domain of the TF;
      ∘ an N-terminal part of a dimerization domain of the TF;
   - the second effector consists of
      ∘ a C-terminal part of a dimerization domain of the TF;
      ∘ an activation domain of the TF;
   - the anti-controller is an N-intein;
   - the third and the fourth effectors are not present.
16. The method according to item 13 or the expression system according to item 13, wherein
   - the controller is a dimerizing transcription factor (TF) modified by insertion of a C-intein;
   - the first effector consists of
      ∘ a DNA-binding domain of the TF;
      ∘ a dimerization domain of the TF;
   - the second effector is an activation domain of the TF;
   - the anti-controller is an N-intein;
   - the third and the fourth effectors are not present.
17. The method according to item 13 or the expression system according to item 13, wherein
   - the controller is a dimerizing transcription factor (TF) modified by insertion of a C-intein;
   - the first effector is a DNA-binding domain of the TF;
   - the second effector consists of
      ∘ a dimerization domain of the TF;
      ∘ an activation domain of the TF;
   - the anti-controller is an N-intein;
   - the third and the fourth effectors are not present.
18. The method according to item 13 or the expression system according to item 13, wherein
   - the controller is an activation domain modified by fusion of a C-intein;
   - the second effector is an activation domain;
   - the first effector is not present;
   - the anti-controller is an N-intein;
   - the third and the fourth effectors are not present;
   - the expression system additionally comprises a co-controller, wherein the co-controller comprises
      ∘ a DNA-binding domain of the TF;
      ∘ a dimerization domain of the TF;
      ∘ an inactive N-intein.
19. A cell comprising the expression system according to any one of the preceding items 2 to 18.

The invention is further illustrated by the following examples and figures, from which further embodiments and advantages can be drawn. These examples are meant to illustrate the invention but not to limit its scope.

### Description of the Figures

- Fig. 1: shows exploiting Intein splicing for building genetic controllers. (a) The basic antithetic integral feedback motif. The arbitrary network to the left represents the regulated network. The input and output molecules of this network are denoted by **X₁** and **X_{L},** respectively. The feedback controller senses the output **X_{L}** and actuates the input **X₁** accordingly. The network in the middle represents the basic antithetic integral feedback motif. The controller molecule **Z₁** is constitutively produced, while **Z₂** is catalytically produced by the output **X_{L}**. Furthermore, **Z₁** and **Z₂** sequester each other to produce an inert complex Ø that has no effect on the closed-loop dynamics. The plot to the right demonstrates that integral controllers achieve Robust Perfect Adaptation (RPA): when a disturbance is injected in the presence of the integral controller, the output concentration level transiently deviates from the desired setpoint but then restores back to it. (b) Possible split-intein reactions. The protein trans-splicing of split inteins is achieved through the specific binding of both intein subunits, which leads to conformational change and chemical bonds shifting on the junction site. The splicing process can be summarized in several essential steps. (i) The precursor 1 containing Intein- N and precursor 2 containing Intein-C are individual proteins. (ii) Intein-N and Intien-C dimerize and reconstitute the active intein domain, which undergoes N-S acyl shift and trans-thioesterification reaction to form a branched intermediate. (iii) The last residue of intein-C forms a succinimide ring, effectively cleaving intein-C from the branched intermediate. (iv) Finally, the end products of protein trans-splicing contain a single-chain protein (comprising the N-terminal of precursor 1 and the C-terminal of precusor2) and a heterodimer (comprising inactive Intein N and Intein-C). Depending on the orientation of the educts (Precursor 1 and Precursor 2), there are several usages of intein-splicing, including sequence ligation, deletion, exchange, or intein cleavage. (c) Examples of realizations of the sequestration reaction via intein splicing. The sequestration reaction lies at the heart of the integral controller shown in panel (a). Based on the capability of split inteins to irreversibly modify the protein sequence or structure, there are several potential implementations to realize the "sequestration reaction" of the antithetic integral controller shown in panel(a). (i) Irreversible Competitive Binding: The heterodimer comprised of the DNA-binding domain and activation domain is irreversibly separated by an intein-mediated removal of intein-N from the DNA-binding domain (ii) Conversion: the target transcription activator is converted to a repressor by an intein-mediated exchange of the activation domain with a repression domain. (iii) Inactivation: the target transcription factor is inactivated by an intein-mediated deletion of its partial sequence. (iv) Degradation: the target transcription factor is destabilized by an intein-mediated ligation of an active degron. (v) Spatial separation/localization: the target transcription factor is removed from the nucleus by intein-mediated exchange of the nuclear localization signal with a nuclear export signal. Observe that, unlike the basic antithetic integral motif where the sequestration reaction annihilates the function of the controller molecules **Z₁** and **Z₂**, these intein-mediated reactions may give rise to one or two products that are functional (for example repressors of gene expression). This feature gives rise to more degrees of freedom in the actuation of the regulated network as compared to the standalone antithetic integral motif.

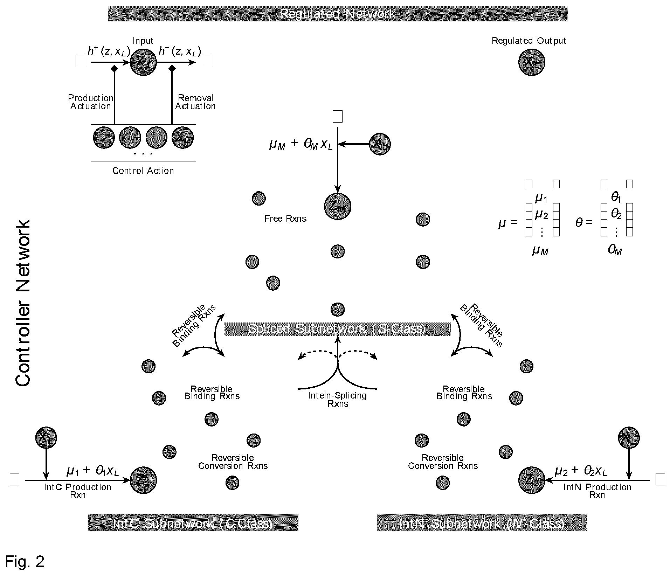

- Fig. 2: shows a general framework for intein-based controllers capable of ensuring robust perfect adaptation. The closed-loop network is comprised of the regulated network that is connected with a feedback controller network. The two networks communicate with each other via the input molecule **X₁** and output molecule **X_{L}** of the regulated network. The end goal of the controller network is to ensure Robust Perfect Adaptation (RPA) for the regulated output molecule **X_{L}**, that is, the controller needs to steer the concentration of the regulated output molecule **X_{L}** to a prescribed value, called the setpoint, despite uncertainties and constant disturbanced in the regulated network. This is done by sensing the levels of the regulated output molecule **X_{L}** and accordingly applying a feedback actuation by producing and/or removing the input molecule **X₁** in a smart autonomous fashion. The setpoint and sensing mechanisms are embedded in the intein production reactions, more precisely in *µ*₁, *µ*₂, *f*₁ and *f*₂. Note that in all the figures, lines with arrowheads indicate activation, lines with T-shaped heads indicate inhibition, and lines with diamond shaped heads can indicate both.

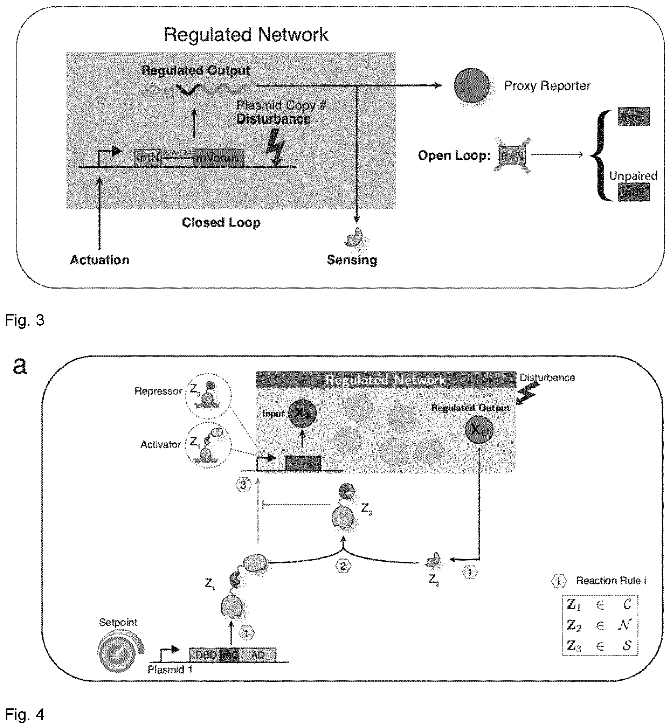

- Fig. 3: shows the description of the regulated network. The regulated network here is a simple transcription process where an mRNA molecule, denoted by **X,** is the regulated output. For the closed-loop circuit, the mRNA molecule is transcribed from a gene that encodes for IntN and a fluorescent protein mVenus separated by a linker. The mRNA is then translated into two separate proteins. The first one is the IntN protein which acts as a sensor capable of driving the feedback controller circuit. The second one is the mVenus fluorescent protein which acts as a proxy reporter enabling the inventors to collect fluorescence measurements. For the open-loop circuit, IntN is replaced with IntC or unpaired IntN which cannot drive the controller feedback circuit and thus breaks the loop. To test for RPA, a disturbance is applied by increasing the plasmid copy numbers via transient transfection.

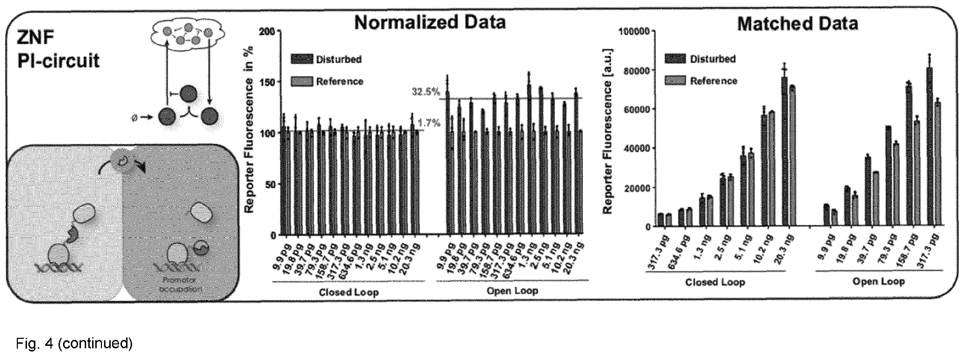

- Fig. 4: shows ZF Circuit. (a) Schematic demonstration of the closed- and open- loop circuits implementing a PI controller using a ZF-based transcription factor. Plasmid 1 here expresses a transcription factor which is comprised of a DBD and an AD separated by an IntC and acts as an activator that initiates the transcription of the mRNA X. When the intein subunits (in molecules **Z₁** and **Z₂**) undergo the intein-splicing reaction, they essentially remove the AD and produce **Z₃** which is comprised of the DBD without the AD and, as a result, acts as a repressor. The setpoint is tuned by the copy number of the Plasmid 1: more plasmids lead to higher fluorescence levels. The classification of the various molecules and reactions are shown in the box and the hexagons, respectively. (b) Experimental Demonstration of RPA. The two bar graphs depict the normalized fluorescent levels (in %) of the reporter at steady-state, acting as a proxy for the regulated output **X,** over a wide range of controller plasmid copy numbers (in pg and ng) that dictate the setpoint. Green bars denote the undisturbed (reference) fluorescence levels; whereas, red bars denote the fluorescence levels after a disturbance is applied by increasing the copy numbers of Plasmid 2. The left bar graph corresponds to the response of the closed-loop circuit while the right one corresponds to the open-loop circuit. Clearly, the closed-loop circuit is capable of rejecting the disturbances over a broad range of setpoints to yield an average error (across all setpoints) of 1.7%, while the open-loop circuit fails to do so.

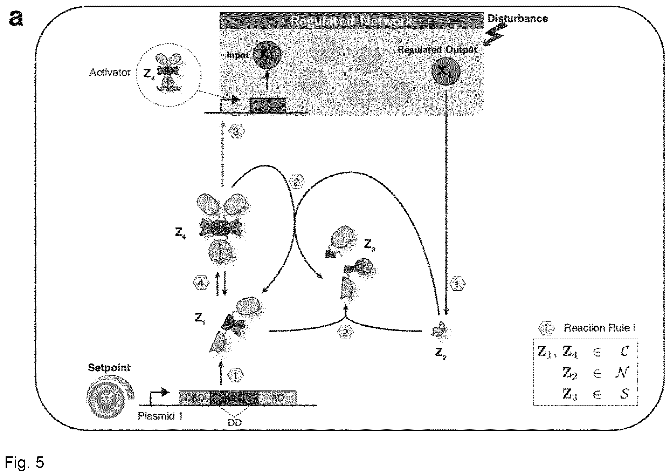

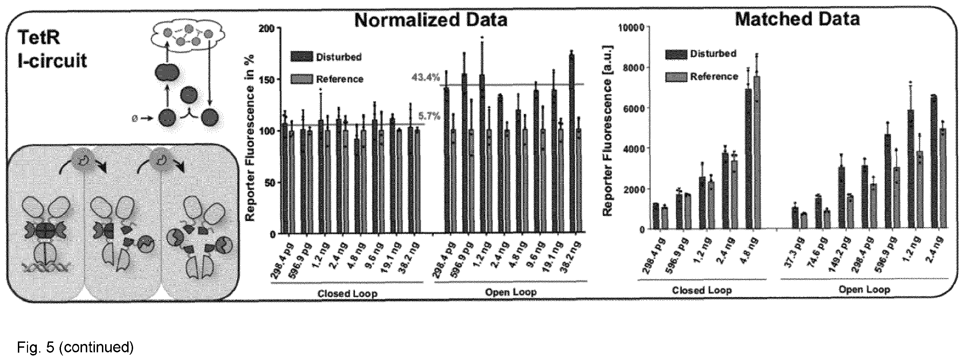

- Fig. 5: shows tetR Circuit. (a) Schematic demonstration of the closed- and open- loop circuits implementing an I controller using a tetR-based transcription factor. Plasmid 1 here expresses a transcription factor which is comprised of a DBD, an AD and a DD broken into two pieces that are separated by an IntC. The expressed transcription factor has to dimerize before it can act as an activator that initiates the transcription of the mRNA X. When the intein subunits in molecules **Z₁** and **Z₂**) undergo an intein-splicing reaction, they essentially separate the two pieces of the DD to yield a complex **Z₃** that has no function. Furthermore, the dimer **Z₄**, having two IntC segments, can also undergo an intein-splicing reaction to give back the monomer **Z₁** and broken complex **Z₃**. The setpoint is tuned by the copy number of Plasmid 1: more controller plasmids lead to higher fluorescence levels. The classification of the various molecules and reactions are shown in the box and the hexagons, respectively. (b) Experimental Demonstration of RPA. The two bar graphs depict the normalized fluorescent levels (in %) of the reporter at steady-state, acting as a proxy for the regulated output **X,** over a wide range of Plasmid 1 copy numbers (in pg and ng) that dictate the setpoint. Green bars denote the undisturbed (reference) fluorescence levels; whereas, red bars denote the fluorescence levels after a disturbance is applied by increasing the copy numbers of Plasmid 2. The left bar graph corresponds to the response of the closed-loop circuit while the right one corresponds to the open-loop circuit. Clearly, the closed-loop circuit is capable of rejecting the disturbances over a broad range of setpoints to yield an average error (across all setpoints) of 5.7%, while the open-loop circuit fails to do so.

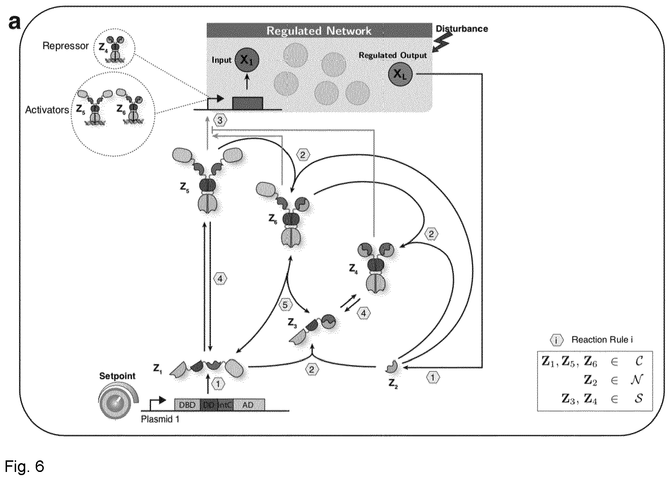

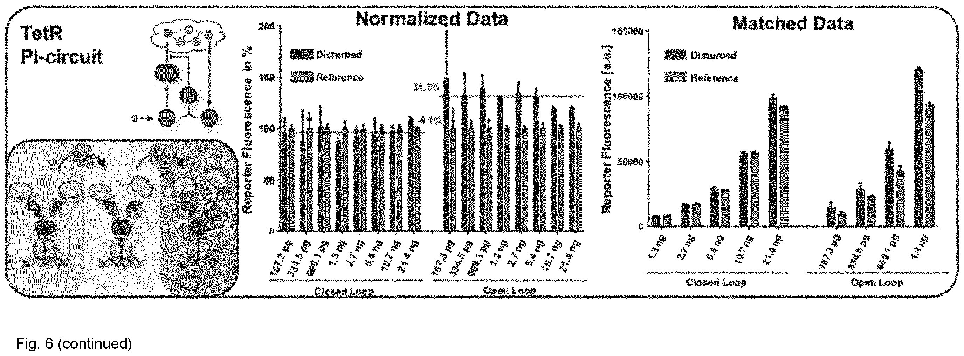

- Fig. 6: shows tTA Circuit. (a) Schematic demonstration of the closed- and open- loop circuits implementing a PI controller using a tTA-based transcription factor. Plasmid 1 here expresses a transcription factor which is comprised of a DBD, DD, IntC and AD. The expressed transcription factor has to dimerize before it can act as an activator that initiates the transcription of the mRNA X. When the intein subunits in molecules **Z₁** and **Z₂**) undergo an intein-splicing reaction, they essentially essentially remove the AD. Without the AD, **Z₃** still has a DD and as a result can still dimerize to produce the dimer **Z₄** that acts as a repressor since it lacks an AD. Note that **Z₁** can either homo-dimerize or hetero-dimerize with **Z₃** to yield **Z₅** and **Z₆** that can act as activators since they have an AD. However, **Z₅** is expected to be a stronger activator than **Z6** since it has an additional AD. Finally, both dimers **Z₅** and **Z₆** have active IntC segments and, as a result, can also undergo intein-splicing reactions with **Z₂**. The setpoint is tuned by the copy number of Plasmid 1: more controller plasmids lead to higher fluorescence levels. The classification of the various molecules and reactions are shown in the box and the hexagons, respectively. (b) Experimental Demonstration of RPA. The two bar graphs depict the normalized fluorescent levels (in %) of the reporter at steady-state, acting as a proxy for the regulated output **X,** over a wide range of Plasmid 1 copy numbers (in pg and ng) that dictate the setpoint. Green bars denote the undisturbed (reference) fluorescence levels; whereas, red bars denote the fluorescence levels after a disturbance is applied by increasing the copy numbers of Plasmid 2. The left bar graph corresponds to the response of the closed-loop circuit while the right one corresponds to the open-loop circuit. Clearly, the closed-loop circuit is capable of rejecting the disturbances over a broad range of setpoints to yield an average error (across all setpoints) of -4.1%, while the open-loop circuit fails to do so.

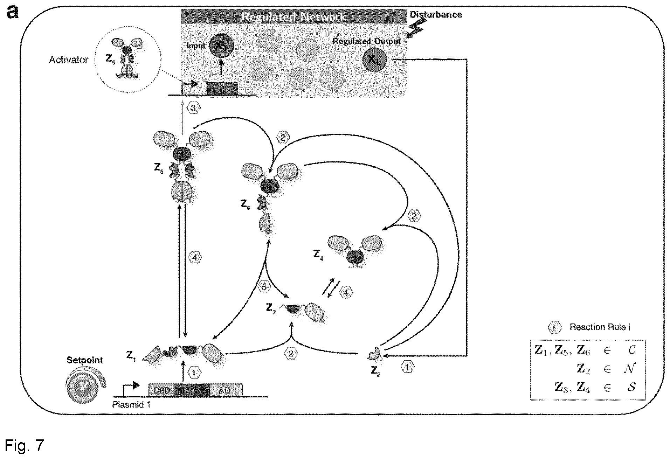

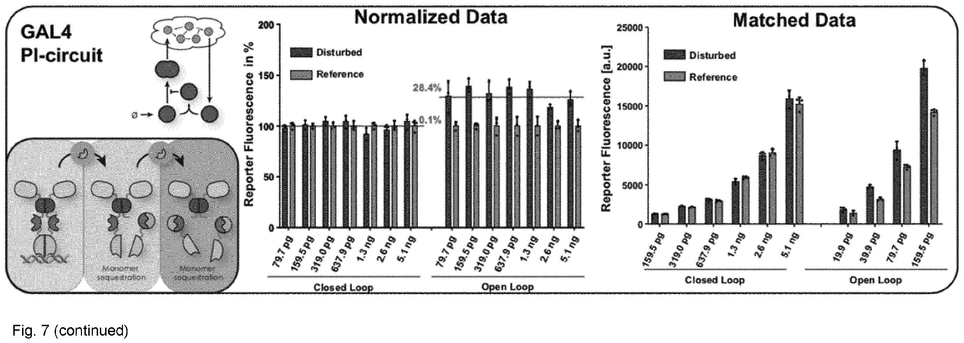

- Fig. 7: shows GAL4 Circuit. (a) Schematic demonstration of the closed- and open- loop circuits implementing a PI controller using a GAL4-based transcription factor. Plasmid 1 here expresses a transcription factor whose domains are the same as those in the tTA circuit of Fig. 6(a), but with only one difference: the order of IntC and the DD is interchanged. This leads to the exact same network topology as that of the tTA circuit of Fig. 6(a), however the molecules **Z₃**, **Z₄** and **Z₆** lack a DBD and thus are incapable of binding to the DNA. As a result, the dimer **Z₅** is the only activator here, and there are no direct repressors. The setpoint is tuned by the copy number of Plasmid 1: more controller plasmids lead to higher fluorescence levels. The classification of the various molecules and reactions are shown in the box and the hexagons, respectively. (b) Experimental Demonstration of RPA. The two bar graphs depict the normalized fluorescent levels (in %) of the reporter at steady-state, acting as a proxy for the regulated output **X,** over a wide range of Plasmid 1 copy numbers (in pg and ng) that dictate the setpoint. Green bars denote the undisturbed (reference) fluorescence levels; whereas, red bars denote the fluorescence levels after a disturbance is applied by increasing the copy numbers of Plasmid 2. The left bar graph corresponds to the response of the closed-loop circuit while the right one corresponds to the open-loop circuit. Clearly, the closed-loop circuit is capable of rejecting the disturbances over a broad range of setpoints to yield an average error (across all setpoints) of 0.1%, while the open-loop circuit fails to do so.

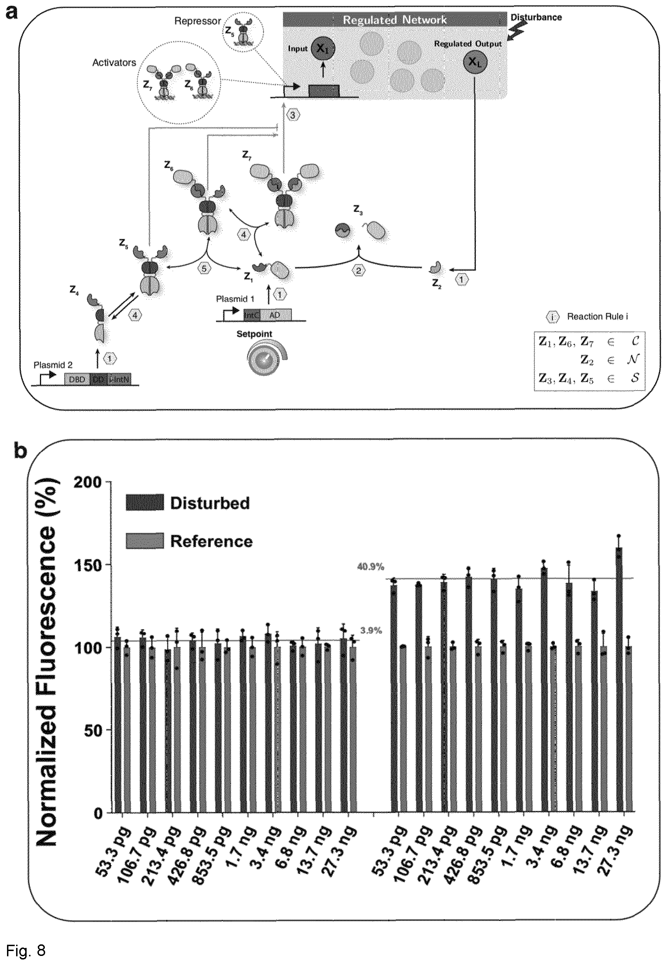

- Fig. 8: shows TetR-Inactive IntN Circuit. (a) Schematic demonstration of the closed- and open- loop circuits implementing an I controller using a transcription factor complex. Plasmid 1 here expresses a fusion protein comprised of IntC and AD. Plasmid 2 here expresses a fusion protein comprised of an inactive IntN and tetR. Two fusion proteins can reversibly heterodimerize through the association between the inactive IntN and IntC and form a functional transcription factor, which acts as an activator that initiates the transcription of the mRNA X. When the intein subunits (in molecules **Z₁** and **Z₂**) undergo the intein-sequestration reaction, they essentially remove the InC from AD in molecule Z1. As a result, AD no longer dimerizes with the DNA binding domain. The setpoint is tuned by the copy number of the Plasmid 1: more plasmids lead to higher fluorescence levels. The classification of the various molecules and reactions are shown in the box and the hexagons, respectively. (b) Experimental Demonstration of RPA. The two bar graphs depict the normalized fluorescent levels (in %) of the reporter at steady-state, acting as a proxy for the regulated output **X,** over a wide range of controller plasmid copy numbers (in pg and ng) that dictate the setpoint. Green bars denote the undisturbed (reference) fluorescence levels; whereas, red bars denote the fluorescence levels after a disturbance is applied by increasing the copy numbers of Plasmid 2. The left bar graph corresponds to the response of the closed-loop circuit while the right one corresponds to the open-loop circuit. Clearly, the closed-loop circuit is capable of rejecting the disturbances over a broad range of setpoints to yield an average error (across all setpoints) of 3.9%, while the open-loop circuit fails to do so.
- Fig. 9: shows a topology of multiple complexes.
- Fig. 10: shows a first topology of multiple Inteins.
- Fig.11: shows a second topology of multiple Inteins.
- Fig. 12: shows a topology of a positive complex.

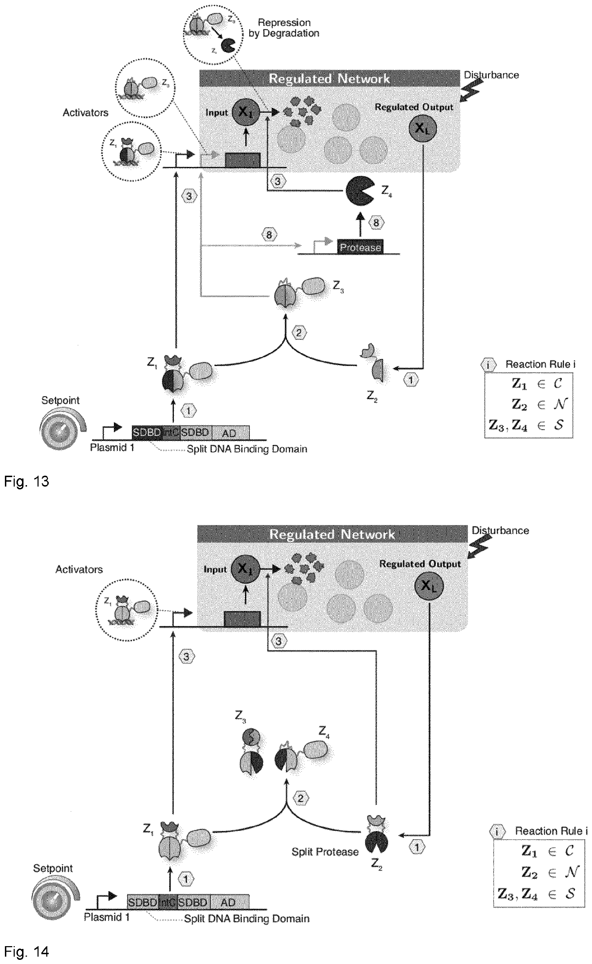

- Fig. 13: shows a topology of a protease.
- Fig. 14: shows a clean version of the regulatory network.

### Examples

### Example 1: General Topologies for Intein-Based Integral Controllers

In this section, the inventors describe a broad class of network topologies that are capable of building intein-based integral controllers that ensure RPA. Consider the general closed-loop network depicted in Fig. 2 where an arbitrary network comprised of L species **X** := ***{*X₁,** . . . , **X_{L}*}*,** referred to as the regulated network, is in a feedback interconnection with the controller network comprised of *M* species **Z := *{Z₁,*** . . . , **Z_{M}*}*.**

The overall objective of the feedback controller is to achieve RPA for the regulated output molecule **X_{L}** by actuating the input molecule **X₁.** The controller network is divided into three subnetworks that belong to one of three classes: the ***N***-class, the **C**-class and the **S**-class. Controller molecules are classified according to the following rules:

### Molecule Rules:

1. All controller molecules containing at least one active IntC belong to the C-class.
2. All controller molecules containing at least one active IntN belong to the N -class.
3. All controller molecules that do not contain any active inteins belong to the S-class.
The various molecules in the three classes react according to the following rules:

### Reaction Rules:

**1.** Productions of controller molecules are either constitutive or driven by the regulated output molecule **X_{L}**
**2.** Any controller molecule in the C-class can undergo an intein-splicing reaction with any controller molecule in the *N*-class to produce at least one product, one of which belongs to the S-class.
**3.** The input molecule **X₁** is either produced or degraded or both by at least one of the controller molecules.
**4.** Within each class, controller molecules are allowed to convert to one another while preserving the number of IntC/IntN.
**5.** Within each class, controller molecules are allowed to reversibly bind to one another while preserving the number of IntC/IntN.
**6.** Controller molecules belonging to the C-class are allowed to reversibly bind with controller molecules belonging to the S-class to produce another controller molecules belonging to the C-class while preserving the number of IntC/IntN.
**7.** Controller molecules belonging to the S-class are allowed to be produced, degraded, and freely react among
**8.** Controller molecules belonging to the S-class are allowed to be produced, degraded, and freely react among each other to yield controller molecules that remain within the S-class.

### Example 2: Intein-Based Genetic PI Controllers

In this section, the inventors present several intein-based controller circuits that are specific embodiments of the general topology presented in the previous section. These embodiments depend on the specific transcription factors used and the insertion location of the intein as well. The inventors begin with the simplest controller circuit and then move on to a higher level of complexity. To experimentally demonstrate that all the presented controllers achieve RPA, the inventors use the same regulated network depicted in Fig. 3 as an example. The objective here is to drive the concentration levels of the regulated output X (measured via the proxy reporter) to a prescribed setpoint, despite the disturbance which is applied here by increasing the plasmid copy numbers as illustrated in Fig. 3.

This RPA property is tested for a wide range of setpoints for all of the controllers presented next. In what follows, let DBD, DD and AD denote the DNA binding domain, dimerization domain and activation domain, respectively.

### Zinc Finger (ZF) Circuit

In this controller circuit, the used transcription factor is based on ZF and is monomeric. That is, it is capable of activating transcription as a monomer (no dimerization reactions). The closed- and open-loop circuits are schematically depicted in Fig. 4(a) where the classification of the various controller molecules and the reaction rules are also shown. The activation by molecule **Z₁** gives rise to an integral controller, while the repression by molecule **Z₃** gives rise to a (filtered) proportional controller and thus summing up to a PI controller. The responses of the circuits depicted in Fig. 4(b) demonstrate that the closed-loop circuit achieves RPA over a broad range of setpoints, while the open-loop circuit fails to do so.

### tetR Circuit

In this controller circuit, the used transcription factor is based on tetR and is dimeric. That is, it is only capable of activating transcription as a dimer. The closed- and open-loop circuits are schematically depicted in Fig. 5(a) where the classification of the various controller molecules and the reaction rules are also shown. The activation by molecule **Z₁** gives rise to an integral controller. The responses of the circuits depicted in Fig. 5(b) demonstrate that the closed-loop circuit achieves RPA over a broad range of setpoints, while the open-loop circuit fails to do so.

### tTA Circuit

In this controller circuit, the used transcription factor is based on tTA and is dimeric. That is, it is only capable of activating transcription as a dimer. The closed- and open-loop circuits are schematically depicted in Fig. 6(a) where the classification of the various controller molecules and the reaction rules are also shown. The activation by molecules **Z₄** and **Z₆** give rise to an integral controller, while the repression by molecule **Z₄** gives rise to (filtered) proportional controller and thus summing up to a PI controller. The responses of the circuits depicted in Fig. 6(b) demonstrate that the closed-loop circuit achieves RPA over a broad range of setpoints, while the open-loop circuit fails to do so.

### GAL4 Circuit

In this controller circuit, the used transcription factor is based on GAL4 and is dimeric. That is, it is only capable of activating transcription as a dimer. The closed- and open-loop circuits are schematically depicted in Fig. 7(a) where the classification of the various controller molecules and the reaction rules are also shown. The activation by molecule **Z₅** gives rise to an integral controller, while the intein-splicing of **Z₅** by **Z₂** acts as a repression mechanism giving rise to a (filtered) proportional controller and thus summing up to a PI controller. The responses of the circuits depicted in Fig. 7(b) demonstrate that the closed-loop circuit achieves RPA over a broad range of setpoints, while the open-loop circuit fails to do so.

### tetR-Inactive IntN Circuit

In this controller circuit, the used transcription factor is based on complex formation between two fusion proteins: inactive IntN fused with a tetR DNA-binding domain and IntC fused with activation domain. That is, it is only capable of activating transcription as a complex. The closed- and open-loop circuits are schematically depicted in Fig. 8(a) where the classification of the various controller molecules and the reaction rules are also shown. The activation by controller molecules **Z₆** and **Z₇** gives rise to an integral controller. The responses of the circuits depicted in Fig. 8(b) demonstrate that the closed-loop circuit achieves RPA over a broad range of setpoints, while the open-loop circuit fails to do so.

### Example 3: Sequences

### Controllers/Co-Controllers:

VP64-IntC(GP41-1)-ZF 
MPKKKRKVGSGEFDALDDFDLDMLGSDALDDFDLDMLGSDALDDFDLDMLGSDALDDFDL
DMLGSGGGGSGGGGSGGGGSATMMLKKILKIEELDERELIDIEVSGNHLFYANDILTHNSN
RGGGGSGGGGSGGGGSGTARPGERPFQCRICMRNFSRQDRLDRHTRTHTGEKPFQCRI
CMRNFSQKEHLAGHLRTHTGEKPFQCRICMRNFSRRDNLNRHLKTHLRGS (SEQ ID NO:
1)
VP64-IntC(NrdJ-1)-ZF 
MPKKKRKVGSGEFDALDDFDLDMLGSDALDDFDLDMLGSDALDDFDLDMLGSDALDDFDL
DMLGSGGGGSGGGGSGGGGSATMGSMEAKTYIGKLKSRKIVSNEDTYDIQTSTHNFFAN
DILVHNSEIGSNRGGGGSGGGGSGGGGSGTARPGERPFQCRICMRNFSRQDRLDRHTRT
HTGEKPFQCRICMRNFSQKEHLAGHLRTHTGEKPFQCRICMRNFSRRDNLNRHLKTHLRG
S (SEQ ID NO: 2)
GAL4(DBD)**_IntC(GP41-1)_**GAL4(DD)-VPR(212) 
MKLLSSIEQACDICRLKKLKCSKEKPKCAKCLKNNWECRYSPKTKRSPLTRAHLTEVESRLE
RLEGSMLKKILKIEELDERELIDIEVSGNHLFYANDILTHNSGSGSQLFLLIFPREDLDMILKM
DSLQDIKALLTGLFVQDNVNKDAVTDRLASVETDMPLTLRQHRISATSSSEESSNKGQRQLT
VSGSSEASGSGRADALDDFDLDMLGSDALDDFDLDMLGSDALDDFDLDMLGSDALDDFD
LDMLINSRSSGSPKKKRKVGSQYLPDTDDRHRIEEKRKRTYETFKSIMKKSPFSGPTDPRP
PPRRIAVPSRSSASVPKPAPQPYPFTSSLSTINYDEFPTMVFPSGQISQASALAPAPPQVLP
QAPAPAPAPAMVSALAQAPAPVPVLAPGPPQAVAPPAPKPTQAGEGTLSEALLQLQFDD
EDLGALLGNSTDPAVFTDLASVDNSEFQQLLNQGIPVAPHTTEPMLMEYPEAITRLVTGAQ
RPPDPAPAPLGAPGLPNGLLSGDEDFSSIADMDFSALLGSGSGSRDSREGMFLPKPEAG
SAISDVFEGREVCQPKRIRPFHPPGSPWANRPLPASLAPTPTGPVHEPVGSLTPAPVPQPL
DPAPAVTPEASHLLEDPDEETSQAVKALREMADTVIPQKEEAAICGQMDLSHPPPRGHLD
ELTTTLESMTEDLNLDSPLTPELNEILDTFLNDECLLHAMHISTGLSIFDTSLFRS (SEQ ID
NO: 3)
tetR**_IntC(GP41-1)_**VP64 
MSRLDKSKVINSALELLNEVGIEGLTTRKLAQKLGVEQPTLYWHVKNKRALLDALAIEMLDRH
HTHFCPLEGESWQDFLRNKAKSFRCALLSHRDGAKVHLGTRPTEKQYETLENQLAFLCQQ
GFSLENALYALSAVGHFTLGCVLEDQEHQVAKEERETPTTDSMPPLLRQAIELFDHQGAEP
AFLFGLELIICGLEKQLKCESGDSGGGGSMLKKILKIEELDERELIDIEVSGNHLFYANDILTH
NSGSGSGRADALDDFDLDMLGSDALDDFDLDMLGSDALDDFDLDMLGSDALDDFDLDMLI
N (SEQ ID NO: 4)
tetR(1-183)**_IntC(GP41-1)_**tetR(184-212)**_VPR** 
MSRLDKSKVINSALELLNEVGIEGLTTRKLAQKLGVEQPTLYWHVKNKRALLDALAIEMLDRH
HTHFCPLEGESWQDFLRNNAKSFRCALLSHRDGAKVHLGTRPTEKQYETLENQLAFLCQQ
GFSLENALYALSAVGHFTLGCVLEDQEHQVAKEERETPTTDSMPPLLRQAIELFDHQGAEP
GSMLKKILKIEELDERELIDIEVSGNHLFYANDILTHNSGSGSAFLFGLELIICGLEKQLKCES
GSPADALDDFDLDMLPADALDDFDLDMLPADALDDFDLDMLPGQPGSSEASGSGRADALD
DFDLDMLGSDALDDFDLDMLGSDALDDFDLDMLGSDALDDFDLDMLINSRSSGSPKKKR
KVGSQYLPDTDDRHRIEEKRKRTYETFKSIMKKSPFSGPTDPRPPPRRIAVPSRSSASVPK
PAPQPYPFTSSLSTINYDEFPTMVFPSGQISQASALAPAPPQVLPQAPAPAPAPAMVSALA
QAPAPVPVLAPGPPQAVAPPAPKPTQAGEGTLSEALLQLQFDDEDLGALLGNSTDPAVFT
DLASVDNSEFQQLLNQGIPVAPHTTEPMLMEYPEAITRLVTGAQRPPDPAPAPLGAPGLP
NGLLSGDEDFSSIADMDFSALLGSGSGSRDSREGMFLPKPEAGSAISDVFEGREVCQPKR
IRPFHPPGSPWANRPLPASLAPTPTGPVHEPVGSLTPAPVPQPLDPAPAVTPEASHLLED
PDEETSQAVKALREMADTVIPQKEEAAICGQMDLSHPPPRGHLDELTTTLESMTEDLNLD
SPLTPELNEILDTFLNDECLLHAMHISTGLSIFDTSLFRS (SEQ ID NO: 5)
TetR-dead IntN 
MSRLDKSKVINSALELLNEVGIEGLTTRKLAQKLGVEQPTLYWHVKNKRALLDALAIEMLDRH
HTHFCPLEGESWQDFLRNNAKSFRCALLSHRDGAKVHLGTRPTEKQYETLENQLAFLCQQ
GFSLENALYALSAVGHFTLGCVLEDQEHQVAKEERETPTTDSMPPLLRQAIELFDHQGAEP
AFLFGLELIICGLEKQLKCESGSGGGGSGGGGTGGGGTGTGYNEVLNVFPKSKKKSYKITL
EDGKEIICSEEHLFPTQTGEMNISGGLKEGMCLYVKEGST (SEQ ID NO: 6)
IntC-VPR

MMLKKILKIEELDERELIDIEVSGNHLFYANDILTHNSGSGSSEASGSGRADALDDFDLDML
GSDALDDFDLDMLGSDALDDFDLDMLGSDALDDFDLDMLINSRSSGSPKKKRKVGSQYL
PDTDDRHRIEEKRKRTYETFKSIMKKSPFSGPTDPRPPPRRIAVPSRSSASVPKPAPQPYP
FTSSLSTINYDEFPTMVFPSGQISQASALAPAPPQVLPQAPAPAPAPAMVSALAQAPAPVP
VLAPGPPQAVAPPAPKPTQAGEGTLSEALLQLQFDDEDLGALLGNSTDPAVFTDLASVDN
SEFQQLLNQGIPVAPHTTEPMLMEYPEAITRLVTGAQRPPDPAPAPLGAPGLPNGLLSGD
EDFSSIADMDFSALLGSGSGSRDSREGMFLPKPEAGSAISDVFEGREVCQPKRIRPFHPP
GSPWANRPLPASLAPTPTGPVHEPVGSLTPAPVPQPLDPAPAVTPEASHLLEDPDEETSQ
AVKALREMADTVIPQKEEAAICGQMDLSHPPPRGHLDELTTTLESMTEDLNLDSPLTPELN
EILDTFLNDECLLHAMHISTGLSIFDTSLFRSTSK (SEQ ID NO: 7)

### Anticontrollers/Open-loop controls + Proxy-Reporter:

IntN(Gp41-1)**_p2A-t2A_**moxVenus 
MGGGGSGGGGTGGGGTGTRSGYCLDLKTQVQTPQGMKEISNIQVGDLVLSNTGYNEVLN
VFPKSKKKSYKITLEDGKEIICSEEHLFPTQTGEMNISGGLKEGMCLYVKEGSGSGATNFSL
LKQAGDVEENPGPGSGEGRGSLLTCGDVEENPGPIDGGSGGSATMVSKGEELFTGVVPIL
VELDGDVNGHKFSVSGEGEGDATYGKLTLKLICTTGKLPVPWPTLVTTLGYGLQCFARYPD
HMKQHDFFKSAMPEGYVQERTIFFKDDGNYKTRAEVKFDGDTLVNRIELKGIDFKEDGNILG
HKLEYNYNSHNVYITADKQKNGIKANFKIRHNIEDGGVQLADHYQQNTPIGDGPVLLPDNHY
LSYQSKLSKDPNEKRDHMVLLEFVTAAGITLGMDELYKGSGSGS (SEQ ID NO: 8)
IntC(Gp41-1)**_p2A-t2A_**moxVenus 
MMLKKILKIEELDERELIDIEVSGNHLFYANDILTHNSGSGSGSGATNFSLLKQAGDVEENPG
PGSGEGRGSLLTCGDVEENPGPIDGGSGGSATMVSKGEELFTGVVPILVELDGDVNGHKF
SVSGEGEGDATYGKLTLKLICTTGKLPVPWPTLVTTLGYGLQCFARYPDHMKQHDFFKSAM
PEGYVQERTIFFKDDGNYKTRAEVKFDGDTLVNRIELKGIDFKEDGNILGHKLEYNYNSHNV
YITADKQKNGIKANFKIRHNIEDGGVQLADHYQQNTPIGDGPVLLPDNHYLSYQSKLSKDPN
EKRDHMVLLEFVTAAGITLGMDELYKGSGSGS (SEQ ID NO: 9)
IntN(NrdJ-1)**_p2A-t2A_**moxVenus 
MNPCCLVGSSEIITRNYGKTTIKEVVEIFDNDKNIQVLAFNTHTDNIEWAPIKAAQLTRPNAEL
VELEIDTLHGVKTIRCTPDHPVYTKNRGYVRADELTDDDELVVAIGSGSGATNFSLLKQAGD
VEENPGPGSGEGRGSLLTCGDVEENPGPIDGGSGGSATMVSKGEELFTGVVPILVELDGD
VNGHKFSVSGEGEGDATYGKLTLKLICTTGKLPVPWPTLVTTLGYGLQCFARYPDHMKQHD
FFKSAMPEGYVQERTIFFKDDGNYKTRAEVKFDGDTLVNRIELKGIDFKEDGNILGHKLEYN
YNSHNVYITADKQKNGIKANFKIRHNIEDGGVQLADHYQQNTPIGDGPVLLPDNHYLSYQSK
LSKDPNEKRDHMVLLEFVTAAGITLGMDELYKGSGSGS (SEQ ID NO: 10)
IntC(NrdJ-1 )_p2A-t2A_moxVenus

MEAKTYIGKLKSRKIVSNEDTYDIQTSTHNFFANDILVHNSEIGSGSGATNFSLLKQAGDVEE
NPGPGSGEGRGSLLTCGDVEENPGPIDGGSGGSATMVSKGEELFTGVVPILVELDGDVNG
HKFSVSGEGEGDATYGKLTLKLICTTGKLPVPWPTLVTTLGYGLQCFARYPDHMKQHDFFK
SAMPEGYVQERTIFFKDDGNYKTRAEVKFDGDTLVNRIELKGIDFKEDGNILGHKLEYNYNS
HNVYITADKQKNGIKANFKIRHNIEDGGVQLADHYQQNTPIGDGPVLLPDNHYLSYQSKLSK
DPNEKRDHMVLLEFVTAAGITLGMDELYKGSGSGS (SEQ ID NO: 11)

### Example 4: Material and Methods

### Data labelling

Illustration of the experimental setup for closed loop characterization is shown in Fig. 3. The controllers were tested in a two-plasmid system. Plasmid 1 encodes the activator with an IntC incorporated under the control of a constitutive output. Plasmid 2 encodes either IntC (in the open loop setting) or IntN (in the closed loop setting), which is fused to the fluorophore mVenus via a P2A-T2A linker. The P2A-T2A leads to ribosome skipping, which leads to the translation of two separate proteins (IntN and mVenus) in a fixed ration from a common mRNA. The mVenus is used as a proxy reporter for the its own mRNA which the controlled output. The gene expression on Plasmid 2 is regulated by the activators expressed from Plasmid 1. In the open loop there is no interaction between the two IntC as they are unable to recognize each other and perform the protein splicing reaction. In the closed loop, the pair can heterodimerize and perform the protein splicing reaction that generates further products, which are able to act on the controlled network. The setpoint is regulated by tuning the constitutive production rate, denoted by µ, via the transfected amount of Plasmid 1. The controller "senses" the abundance of the controlled output via the translation of the mRNA encoded in Plasmid 2, whose rate is denoted by θ. Furthermore, Plasmid 2 is used for disturbances as its concentration does not alter the setpoint which is equal to µ/θ. The amount of transfected Plasmid 2 is fixed to a certain value for all setpoints and the output is regarded as the reference. The same experiment is repeated for all setpoints with a higher amount of Plasmid 2 and is regarded as the disturbed output.

A simplified schematic of the controller topology can be seen on the left panel of Figs. 4-7 together with a selected subset of the controller species. A green (resp. red) background indicates upregulation (resp. downregulation) of the output concentration; whereas, a grey background indicates no impact on the output concentration. The middle panel shows the normalized fluorescent data of the proxy reporter. The disturbed and reference triplicate measurements were normalized to the mean fluorescence of the reference data for every setpoint. The X-axis is grouped to correspond to the closed- and open-loop circuits, and shows the amount of Plasmid 1 transfected within every well. The y-axis shows the normalized fluorescent output. The red horizontal line gives the normalized output averaged over all setpoints. The labeling above the lines indicates the average error in % compared to the reference data. The right panel shows the non-normalized data over a selected subset of setpoint that match in fluorescence between the open- and the closed-loop circuits.

### Plasmid Construction

All plasmids were generated with a mammalian adaptation of the modular cloning (MoClo) yeast toolkit standard. All individual parts were generated by PCR amplification (Phusion Flash High-Fidelity PCR Master Mix; Thermo Scientific) or synthesized with Twist Bioscience. The parts were then assembled with golden gate assembly. All enzymes for plasmid construction were obtained from New England Biolabs (NEB). Constructs were chemically transformed into *E. coli* Top10 strains.

### Cell culture

All experiments were performed with HEK293T cells (ATCC, strain number CRL-3216). The cells were cultured in Dulbecco's modified Eagle's medium (DMEM; Gibco) supplemented with 10 % FBS (Sigma-Aldrich), 1x GlutaMAX (Gibco), 1 mm Sodium Pyruvate (Gibco), penicillin (100 U/µL), and streptomycin (100 µg/mL) (Gibco) at 37°C with 5% CO2. The cell culture was passaged into a fresh T25 flask (Axon Lab) every 2 to 3 days. Upon detachment some part of the cell suspension was used for the transfection.

### Transfection

All plasmids were isolated using ZR Plasmid Miniprep-Classic (Zymo Research). The plasmids were introduced to the HEK293T cells via suspension transfection. A transfection solution in Opti-MEM I (Gibco) was prepared using Polyethylenimine (PEI) "MAX" (MW 40000; Polysciences, Inc.) at a 1:3 (µg DNA to µg PEI) ratio while the culture was detached with Trypsin-EDTA (Gibco). The cell density was assessed with the automated cell counter Countess II FL (Invitrogen). 100 µL of culture with 26000 cells was transferred in each well of the plate Nunc Edge 96-well plate (Thermo Scientific). The transfection mixture was added to the cells once it has incubated for approximately 30 min.

### Flow cytometry

The cells were detached approximately 48 hours after transfection on the Eppendorf ThermoMixer C at 25°C at 700 rpm with 53 µL Accutase solution (Sigma-Aldrich) per well for 20 min. The fluorescence data was collected on the Beckman Coulter CytoFLEX S flow cytometer with the 488 nm excitation with a 525/40+OD1 bandpass filter and the 638 nm excitation with a 660/10 bandpass filter. All data was processed with the CytExpert 2.3 software.

### Cited prior art documents:

1. C. Kemmer, M. Gitzinger, M. Daoud-EI Baba, V. Djonov, J. Stelling, and M. Fussenegger, "Self-sufficient control of urate homeostasis in mice by a synthetic circuit," Nature biotechnology, vol. 28, no. 4, p. 355, 2010.
2. K. Rössger, G. Charpin-EI-Hamri, and M. Fussenegger, "A closed-loop synthetic gene circuit for the treat- ment of diet-induced obesity in mice," Nature communications, vol. 4, no. 1, pp. 1-9, 2013.
3. M. Xie, H. Ye, H. Wang, G. Charpin-EI Hamri, C. Lormeau, P. Saxena, J. Stelling, and M. Fussenegger, "β- cell-mimetic designer cells provide closed-loop glycemic control," Science, vol. 354, no. 6317, pp. 1296-1301, 2016.
4. F. Xiao and J. C. Doyle, "Robust perfect adaptation in biomolecular reaction networks," in 2018 IEEE Conference on Decision and Control (CDC). IEEE, 2018, pp. 4345-4352.
5. M. H. Khammash, "Perfect adaptation in biology," Cell Systems, vol. 12, no. 6, pp. 509-521, 2021.
6. B. A. Francis and W. M. Wonham, "The internal model principle of control theory," Automatica, vol. 12, no. 5, pp. 457-465, 1976.
7. C. Briat, A. Gupta, and M. Khammash, "Antithetic integral feedback ensures robust perfect adaptation in noisy biomolecular networks," Cell Systems, vol. 2, no. 1, pp. 15-26, 2016. [Online]. Available: https://doi.org/10.1016/j.cels.2016.01.004
8. M. Filo, S. Kumar, and M. Khammash, "A hierarchy of biomolecular proportional-integral-derivative feed- back controllers for robust perfect adaptation and dynamic performance," Nature Communications, vol. 13, no. 1, pp. 1-19, 2022.
9. S. K. Aoki, G. Lillacci, A. Gupta, A. Baumschlager, D. Schweingruber, and M. Khammash, "A universal biomolecular integral feedback controller for robust perfect adaptation," Nature, p. 1, 2019.
10. T. Frei, C.-H. Chang, M. Filo, A. Arampatzis, and M. Khammash, "Genetically engineered proportional-integral feedback controllers for robust perfect adaptation in mammalian cells," bioRxiv, 2021. [Online]. Available: https://www.biorxiv.org/content/early/2021/08/13/2020.12.06.412304

## Claims

1. A method for constant-level expression of an output, said method comprising the steps:
a. providing a cell or a cell-free system, wherein the cell or the cell-free system is capable of expressing a gene encoding the output;
b. inserting an expression system into said cell or cell-free system,
wherein the expression system comprises:
i. a gene encoding a controller, wherein the controller comprises:
I. an optional first effector;
II. a first split-intein; and
III. an optional second effector;
ii. a gene encoding an anti-controller, wherein the anti-controller comprises:
I. an optional third effector;
II. a second split-intein; and
III. an optional fourth effector;
wherein
- at least one of the optional effectors is present;
- one of the first split-intein and the second split-intein is an N-intein and the other one is a C-intein;
- the controller and the anti-controller are capable of undergoing a trans-splicing reaction via the steps:
- excising the first split-intein from the controller, and excising the second split-intein from the anti-controller, and
a) if the first split-intein is an N-intein, and the second split-intein is a C-intein:
- rejoining
∘ the first effector if present with
∘ the fourth effector if present
yielding a first splice-product;
- assembling a protein complex of
∘ the N-intein
▪ bound to the second effector if present
with
∘ the C-intein
▪ bound to the third effector if present
yielding a second splice-product;
b) if the first split-intein is a C-intein, and the second split-intein is an N-intein:
- rejoining
∘ the second effector if present with
∘ the third effector if present
yielding a first splice-product;
- assembling a protein complex of
∘ the N-intein
▪ bound to the fourth effector if present
with
∘ the C-intein
▪ bound to the first effector if present
yielding a second splice-product;
wherein at least the second splice-product is yielded;
- the anti-controller is capable of downregulating the controller stoichiometrically via undergoing the trans-splicing reaction;
- the second splice-product and the optional first splice-product have a weaker, neutral, or opposite effect on the concentration of the output in comparison to the effect of the controller;
- constant-level expression of the output is achieved via a route selected from the group consisting of:
(1)
- the controller upregulates the concentration of the output;
- the output upregulates the concentration of the anti-controller;
(2)
- the controller downregulates the concentration of the output;
- the output downregulates the concentration of the anti-controller;
(3)
- the controller upregulates the concentration of the output;
- the output downregulates the concentration of the controller;
(4)
- the controller downregulates the concentration of the output;
- the output upregulates the concentration of the controller;
c. exposing said cell or cell-free system to a condition, wherein the expression level of the output is perturbed from a target level to a perturbed level;
d. equilibration of the expression level of the output back to the target level;
wherein the first, second, third, and fourth effector are peptides or polypeptides, and the first and second intein, the controller, and the anti-controller are polypeptides, and the output is a peptide, a polypeptide, or an mRNA.

2. An expression system for constant-level expression of an output, said system comprising:
a. a gene encoding a controller, wherein the controller comprises:
i.) an optional first effector;
ii.) a first split-intein; and
iii.) an optional second effector;
b. a gene encoding an anti-controller, wherein the anti-controller comprises:
i.) an optional third effector;
ii.) a second split-intein; and
iii.) an optional fourth effector;
wherein
- at least one of the optional effectors is present;
- one of the first split-intein and the second split-intein is an N-intein and the other one is a C-intein;
- the controller and the anti-controller are capable of undergoing a trans-splicing reaction via the steps:
- excising the first split-intein from the controller, and excising the second split-intein from the anti-controller, and
a) if the first split-intein is an N-intein, and the second split-intein is a C-intein:
- rejoining
∘ the first effector if present with
∘ the fourth effector if present
yielding a first splice-product;
- assembling a protein complex of
∘ the N-intein
▪ bound to the second effector if present
with
∘ the C-intein
▪ bound to the third effector if present
yielding a second splice-product;
b) if the first split-intein is a C-intein, and the second split-intein is an N-intein:
- rejoining
∘ the second effector if present with
∘ the third effector if present
yielding a first splice-product;
- assembling a protein complex of
∘ the N-intein
▪ bound to the fourth effector if present
with
∘ the C-intein
▪ bound to the first effector if present
yielding a second splice-product;
wherein at least the second splice-product is yielded;
- the anti-controller is capable of downregulating the controller stoichiometrically via undergoing the trans-splicing reaction;
- the second splice-product and the optional first splice-product have a weaker, neutral, or opposite effect on the concentration of the output in comparison to the effect of the controller;
- a route via which constant-level expression of the output is achieved is selected from the group consisting of:
(1)
- the controller is capable of upregulating the concentration of the output;
- the output is capable of upregulating the concentration of the anti-controller;
(2)
- the controller is capable of downregulating the concentration of the output;
- the output is capable of downregulating the concentration of the anti-controller;
(3)
- the controller is capable of upregulating the concentration of the output;
- the output is capable of downregulating the concentration of the controller;
(4)
- the controller is capable of downregulating the concentration of the output;
- the output is capable of upregulating the concentration of the controller,
wherein the first, second, third, and fourth effector are peptides or polypeptides, and the first and second intein, the controller, and the anti-controller are polypeptides, and the output is a peptide, a polypeptide, or an mRNA.

3. The method according to claim 1 or the expression system according to claim 2, wherein the controller comprises a further first intein and/or the anti-controller comprises a further second intein and the trans-splicing reaction is executed multiple times finally yielding the second splice-product and optionally the first splice-product.

4. The method according to any one of the preceding claims 1 or 3 or the expression system according to any one of the preceding claims 2 to 3, wherein for case (1) that
• the controller is capable of upregulating the concentration of the output;
• the output is capable of upregulating the concentration of the anti-controller;
additionally, the anti-controller is capable of downregulating the concentration of the output.

5. The method according to any one of the preceding claims 1 or 3 or the expression system according to any one of the preceding claims 2 to 3, wherein for case (2) that
• the controller is capable of downregulating the concentration of the output;
• the output is capable of downregulating the concentration of the anti-controller;
additionally, the anti-controller is capable of upregulating the concentration of the output.

6. The method according to any one of the preceding claims 1 or 3 or the expression system according to any one of the preceding claims 2 or 3, wherein for case (3) that
• the controller is capable of upregulating the concentration of the output;
• the output is capable of downregulating the concentration of the controller;
additionally, the anti-controller is capable of downregulating the concentration of the output.

7. The method according to any one of the preceding claims 1 or 3 or the expression system according to any one of the preceding claims 2 or 3, wherein for case (4) that
• the controller is capable of downregulating the concentration of the output;
• the output is capable of upregulating the concentration of the controller;
additionally, the anti-controller is capable of upregulating the concentration of the output.

8. The method according to any one of the preceding claims 1 or 3 to 7 or the expression system according to any one of the preceding claims 2 to 7, wherein the first intein is inserted into the controller in a loop region of the first and second effector and/or the second intein is inserted into the anti-controller in a loop region of the third and fourth effector.

9. The method according to any one of the preceding claims 1 or 3 to 8 or the expression system according to any one of the preceding claims 2 to 8, wherein
• the first effector, and/or
• the second effector, and/or
• the third effector, and/or
• the fourth effector
comprise a domain of a transcription factor.

10. The method according to claim 9 or the expression system according to claim 9, wherein
• the controller is a non-dimerizing transcription factor (TF) modified by insertion of a C-intein;
• the first effector is a DNA-binding domain of the TF;
• the second effector is an activation domain of the TF;
• the anti-controller is an N-intein;
• the third and the fourth effectors are not present.

11. The method according to claim 9 or the expression system according to claim 9, wherein
• the controller is a dimerizing transcription factor (TF) modified by insertion of a C-intein;
• the first effector consists of
∘ a DNA-binding domain of the TF;
∘ an N-terminal part of a dimerization domain of the TF;
• the second effector consists of
∘ a C-terminal part of a dimerization domain of the TF;
∘ an activation domain of the TF;
• the anti-controller is an N-intein;
• the third and the fourth effectors are not present.

12. The method according to claim 9 or the expression system according to claim 9, wherein
• the controller is a dimerizing transcription factor (TF) modified by insertion of a C-intein;
• the first effector consists of
∘ a DNA-binding domain of the TF;
∘ a dimerization domain of the TF;
• the second effector is an activation domain of the TF;
• the anti-controller is an N-intein;
• the third and the fourth effectors are not present.

13. The method according to claim 9 or the expression system according to claim 9, wherein
• the controller is a dimerizing transcription factor (TF) modified by insertion of a C-intein;
• the first effector is a DNA-binding domain of the TF;
• the second effector consists of
∘ a dimerization domain of the TF;
∘ an activation domain of the TF;
• the anti-controller is an N-intein;
• the third and the fourth effectors are not present.

14. The method according to claim 9 or the expression system according to claim 9, wherein
• the controller is an activation domain modified by fusion of a C-intein;
• the second effector is an activation domain;
• the first effector is not present;
• the anti-controller is an N-intein;
• the third and the fourth effectors are not present;
• the expression system additionally comprises a co-controller, wherein the co-controller comprises
∘ a DNA-binding domain of the TF;
∘ a dimerization domain of the TF;
∘ an inactive N-intein.

15. A cell comprising the expression system according to any one of the preceding claims 2 to 14.
